# EUROPEAN PATENT SPECIFICATION

(11) **EP 4 102 974 B1**
(45) Date of publication and mention of the grant of the patent: **23.10.2024**
(21) Application number: 21705923.7
(22) Date of filing: 12.02.2021
(51) Int. Cl.: C12N 9/26, C12N 9/34, A21D 8/04, A21D 13/062

(54) **A PROCESS FOR THE PRODUCTION OF A BAKED PRODUCT WITHOUT ADDITION OF SUGAR**
VERFAHREN ZUR HERSTELLUNG EINES GEBACKENEN PRODUKTES OHNE ZUSATZ VON ZUCKER
PROCÉDÉ DE PRODUCTION D'UN PRODUIT CUIT SANS AJOUT DE SUCRE

(30) Priority: 14.02.2020 DK PA202070088
(43) Date of publication of application: 21.12.2022
(73) Proprietor: LANTMÄNNEN UNIBAKE HOLDING A/S, 2300 Copenhagen S (DK)
(72) Inventor: MÖLLER, Rune Gerner, 3480 Fredensborg (DK)
(74) Representative: Aera A/S
(86) International application number: PCT/EP2021/053474
(87) International publication number: WO 2021/160812

(56) References cited:
- WO-A1-2014/161876
- WO-A1-2016/005452
- WO-A1-2019/238423
- WO-A1-2020/182879
- WO-A2-2020/086430
- US-A1- 2018 177 202
- H.B. ADITIYA ET AL: "Second generation bioethanol production: A critical review", RENEWABLE AND SUSTAINABLE ENERGY REVIEWS, vol. 66, 1 December 2016 (2016-12-01), US, pages 631 - 653, XP055503053, ISSN: 1364-0321, DOI: 10.1016/j.rser.2016.07.015

## Description

### FIELD

The present invention relates to a process for preparing a baked product that is made without any addition of sugar. The baked product has still a suitable sweetness intensity and a good taste. The baked product has a very low content of fructose and, thus, it may be suitable for use also for fructose-intolerant humans. The baked product is obtained by a two-step enzymatic process involving a first step of providing sugar from starch, suitable for a fermentation process, followed by a second step of enzymatic hydrolysis of polysaccharides, oligosaccharides and disaccharides, notably to form primarily glucose and maltose. The novel process further allows for an optimized and/or shortened proofing of the dough in the first step. The shortened proofing time is advantageous as it reduces the total time it takes for the production of the baked product. The present invention also relates to a cocktail of three enzymes, which is a combination consisting of an alpha-amylase (EC 3.2.1.1), which is thermo-labile and has activity in a range of from 30 to 65 °C, an amyloglucosidase (EC 3.2.1.3), which is thermo-stable and has optimum activity at about 60-65 °C, and a maltogenic amylase (EC 3.2.1.133), which has optimal activity in a range of from 50 to 75 °C, and wherein the thermo-labile alpha-amylase, the thermo-stable amyloglucosidase and the maltogenic amylase are present in ratios corresponding to from 20 to 48 Fau or from 28 to 40 Fau of the thermo-labile alpha-amylase, from 578 to 1650 Agu or from 660 to 1650 Agu of the thermo-stable amyloglucosidase, and from 500 to 2500 Manu or from 1000 to 2500 Manu of the maltogenic amylase.

### BACKGROUND

Due to health issues, sugar consumption has become very high on the health agenda. Thus, within EU, there is a target to reduce added sugars by a minimum of 10% by 2020 in different food applications. A lot of sugar replacement ingredients have been developed in the last years to avoid addition of sugar to food, including artificial sweeteners (polyols), natural sweeteners (inulin, oligofructose), which are mainly hydrolyzed by acids or by enzymes.

Besides the health issues, there is a general interest in keeping the content of added sugar in baked product as low as possible. The reasons include reducing the cost price of the baked product, avoiding impact of fluctuations in the price of sugar, obtaining the possibility of reducing the amount of salt added, etc. Also, there is a growing general skepticism against synthetic or perceived un-natural additives in the consumer group.

Baked products with a reduced or without a content of added sugar are already known:
WO 2016/005452 (Purac Biochem BV) describes products formed from dough comprising a thermally stable amyloglucosidase, and a raw starch degrading amyloglycosidase and/or an anti-staling amylase, which may be a maltogenic amylase. It is stated that the level of added sugar included in the dough can be substantially reduced, and even eliminated, while still achieving a sweet product.
WO 91/01088 (Kama Danish Pastry A/S) describes a method for preparing a frozen yeast dough, wherein the dough is prepared from flour, water, yeast and one or more amylases and possibly other conventional dough ingredients.
WO 2013/028071 (CSM Nederland B.V.) describes the use of an anti-staling enzyme mixture consisting of a maltogenic amylase and an amyloglucosidase.
CN 101461392 relates to a sugar-free bread made from high wheat gluten, low wheat gluten, anhydrous butter, sugar-free modifying agent, sugar-free milk powder, bread improver, yeast, egg. It may contain a fungal alpha-amylase.
WO 2019/238423 (Novozymes A/S) describes a method for producing a dough with a reduced amount of sugar comprising adding a raw starch degrading alpha-amylase (GH13_1 amylase), a glucoamylase and an alpha-amylase to dough ingredients.
US 2018/0177202 (blareau et al.) describes a breadmaking improver comprising maltogenic exoamylase, amyloglucosidase, alpha-amylase and xylanase. However, no details regarding the properties of the enzymes are given.

Still, none of the processes demonstrated in the prior art i) shows that it is possible to significantly reduce the proofing time and ii) results in a baked product that is as paltable as a traditional baked product having been baked with added sugar and which has a low content of individual mono- or disaccharides and a low total content of mono- and di-saccharides.

### DETAILED DESCRIPTION

### Process for producing a product according to the invention

1. In its broadest aspect, the invention relates to a process for producing a bakery product with no-added sugar, the process comprising
   i) mixing flour with a content of damaged starch of at least 5% by weight with a combination consisting of a thermo-labile alpha-amylase (EC 3.2.1.1), an amyloglucosidase (EC 3.2.1.3) and a maltogenic amylase (EC 3.2.1.133); a yeast; water, and optionally other ingredients common for preparing a dough, to obtain a dough,
   ii) proofing the dough,
   iii) optionally shaping the dough into a desired form,
   iv) optionally, infreezing the un-baked product at a temperature in a range of from -35 °C - -45 °C for a time period of from about 15 min to about 30 min followed by frezzing at about -18 °C, and
   v) optionally pre-baking or baking the optionally shaped dough at a temperature in a range of from 180 to 250°C,
   wherein the combination in step i) is a combination consisting of an alpha-amylase (EC 3.2.1.1), which is thermo-labile and has activity in a range of from 30 to 65 °C, an amyloglucosidase (EC 3.2.1.3), which is thermo-stable and has optimum activity at about 60-65 °C, and a maltogenic amylase (EC 3.2.1.133), which has optimal activity in a range of from 50 to 75 °C, and wherein the thermo-labile alpha-amylase, the thermo-stable amyloglucosidase and the maltogenic amylase are present in ratios corresponding to
   from 20 to 48 Fau or from 28 to 40 Fau of the thermo-labile alpha-amylase,
      from 578 to 1650 Agu or from 660 to 1650 Agu of the thermo-stable amyloglucosidase, and
   from 500 to 2500 Manu or from 1000 to 2500 Manu of the maltogenic amylase.

In the following, the combination of the three enzymes in step i) is referred to as a cocktail of three enzymes.

The present invention also relates to this cocktail of three enzymes, namely to a combination consisting of an alpha-amylase (EC 3.2.1.1), which is thermo-labile and has activity in a range of from 30 to 65 °C, an amyloglucosidase (EC 3.2.1.3), which is thermo-stable and has optimum activity at about 60-65 °C, and a maltogenic amylase (EC 3.2.1.133), which has optimal activity in a range of from 50 to 75 °C, and wherein the thermo-labile alpha-amylase, the thermo-stable amyloglucosidase and the maltogenic amylase are present in ratios corresponding to
from 20 to 48 Fau or from 28 to 40 Fau of the thermo-labile alpha-amylase,
   from 578 to 1650 Agu or from 660 to 1650 Agu of the thermo-stable amyloglucosidase, and
from 500 to 2500 Manu or from 1000 to 2500 Manu of the maltogenic amylase.

In embodiments, the present invention relates to a process for producing a baked product with no-added sugar, the process comprising
i) mixing flour with a content of damaged starch of at least 5% w/w such as in a range of from 7% to 9% by weight with said cocktail of three enzymes ; a yeast; water and optionally other ingredients common for preparing a dough, to obtain a dough,
ii) proofing the dough,
iii) baking the dough at a temperature in a range of from 180 to 250°C.

In embodiments, the present invention also relates to a process for producing a baked product with no-added sugar, the process comprising
i) mixing flour with a content of damaged starch of at least 5% w/w such as in a range of from 7% to 9% by weight with said cocktail of three enzymes ; a yeast; water and optionally other ingredients common for preparing a dough, to obtain a dough,
ii) proofing the dough,
iii) pre-baking the dough to a degree of from 70% to 85% such as 80% at a temperature in a range of from 180 to 250°C.

In embodiments, the present invention also relates to a process for producing an un-baked product with no-added sugar. In such cases, the consumer will buy the un-baked product in frozen form and arrange for the final baking. Thus, the process comprises
i) mixing flour with a content of damaged starch of at least 5% w/w such as in a range of from 7% to 9% by weight with said cocktail of three enzymes; a yeast; water and optionally other ingredients common for preparing a dough, to obtain a dough,
ii) proofing the dough,
iii) shaping the dough into an un-baked product,
iv) infreezing the un-baked product at a temperature in a range of from -35 °C - -45 °C for a time period of from aobut 15 min to about 30 min followed by frezzing at about -18 °C , and
v) optionally, pre-baking or baking the frozen un-baked product at a temperature in a range of from 180 to 250°C.

In aspects of the invention, the pre-baking step is included in the process. In other aspects both the baking step is included in the process.

Thus, in its broadest aspect, the present invention relates to a process for producing a product with no-added sugar, the process comprising
i) mixing flour with a content of damaged starch of at least 5% w/w such as in a range of from 7% to 9% by weight with said cocktail of three enzymes ; a yeast; water and optionally other ingredients common for preparing a dough, to obtain a dough,
ii) proofing the dough,
iii) optionally, shaping the dough into the desired form,
iv) optionally, infreezing the un-baked product at a temperature in a range of from -35 °C - -45 °C for a time period of from aobut 15 min to about 30 min followed by frezzing at about -18 °C, and
v) optionally pre-baking or baking the optionally shaped dough at a temperature in a range of from 180 to 250°C.

In aspects of the invention, steps i), ii), and v) are included, where step v) is a baking step the final product is a baked product).

In aspects of the invention, steps i), ii), and v) are included, where step v) is a pre-baking step the final product is a pre-baked product).

In aspects of the invention, steps i), ii), and v) are included, where step v) is a pre-baking step followed by step iv) (the final product is a frozen, pre-baked product).

In other aspects of the invention, steps i), ii), iii) and v) are included, where step v) is a baking step (the final product is a baked, shaped product).

In other aspects of the invention, steps i), ii), and iv) are included (the final product is a proofed, frozen, un-baked product).

In other aspects of the invention, steps i), ii), iii) and iv) are included (the final product is a proofed, shaped, frozen, un-baked product).

Described herein are also the products obtained by the processes described above (not part of the claimed invention).

Important features of a process according to the invention are a) no addition of sugar and b) the use of a two-step enzymatic process.

In the present context the terms "no addition of sugar" and "with no-added sugar" mean that none of the ingredients used in the production of a baked product according to the present invention is a sugar in the form of a monosaccharide such as e.g. glucose, fructose, or a disaccharide such as e.g. maltose or saccharose, i.e. there is no external addition of sugar in the baking process. Other ingredients, such as flour may contain oligosaccharides or polysaccharides that enzymatically can be degraded to mono- or disaccharides and they may contain a minor amount of a mono- or disaccharide, such as about 1-2% by weight of eg glucose, fructose, sucrose and raffinose. However, a person skilled in the art will know that the amounts of mono-, di-, oligo- and polysaccharides in a flour may vary dependent on the particular flour used.

One of the challenges in restraining from adding sugar to the dough is how to obtain sufficient and optimized proofing of the dough. Proofing of the dough is normally obtained by a fermentation process; whereby yeast organisms consume sugar in the dough and produce ethanol and carbon dioxide as waste products. The carbon dioxide forms bubbles in the dough and expands it (proofing).

When no sugar is added to the dough as in a process of the invention, another mechanism must apply. To this end, it is important that the flour contains a certain amount of damaged starch. Damaged starch refers to the portion of kernel starch that has been physically broken or fragmented during milling. Damaged starch is believed to have a strong influence on the dough and baking process. In the present process, damaged starch is a suitable substrate for the alpha-amylase to provide the necessary sugar molecules that are required for the fermentation process by the yeast. It is envisaged that the alpha-amylase (most likely with some contribution from the combination of amyloglucosidase and maltogenic amylase) relatively fast produces the necessary sugars and in a sufficient amount in to order to obtain fast proofing. It is assumed that the sugars formed are consumed approximately at the same time as they are formed. This is supported by the observation done by the inventors that the proofing time is reduced for dough with no-added sugar compared to dough with sugar added.

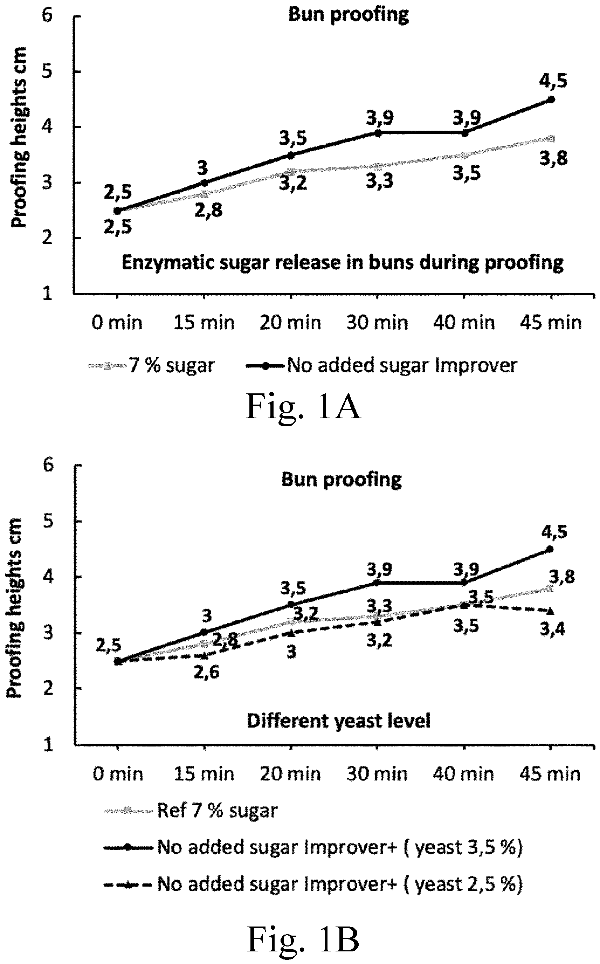

Figure 1A and 1B show that the proofing step is controlled either with yeast or with proofing time, or both. The upper graph in both figures relates to a product without any sugar added. The other graphs relate to products having 7% sugar added. In order to obtain e.g. a proofing height of 3.5 cm, the proofing time can be reduced from the proofing time from 40 min to 20 min, thus, in general a 50% reduction in proofing time. Moreover, the choice and content of yeast can control the proofing stage and volume. In the examples Danish standard yeast (Malteser yeast) has been used. In general, a reduction in proof time of from 25 to 30% is obtained. Experimental details are given in Example 4.

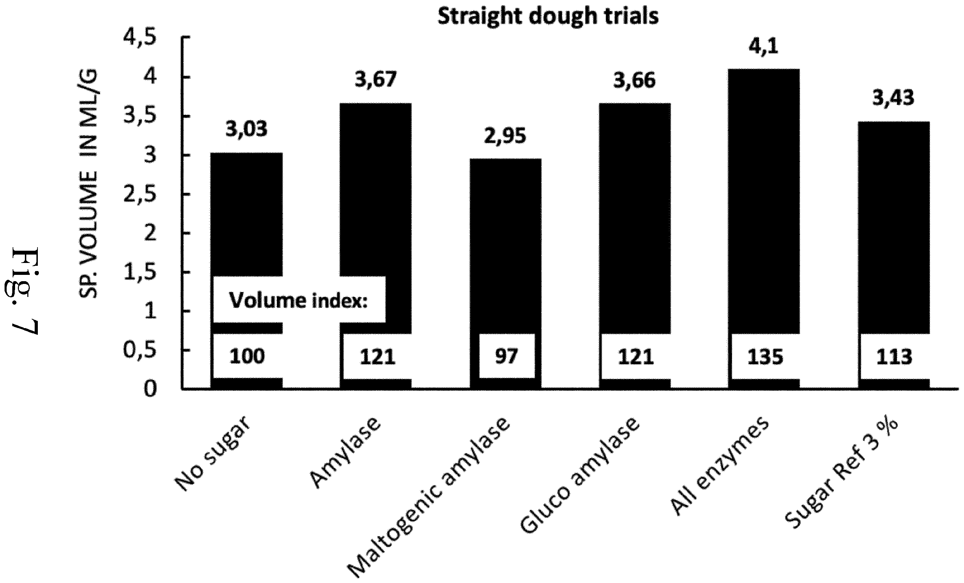

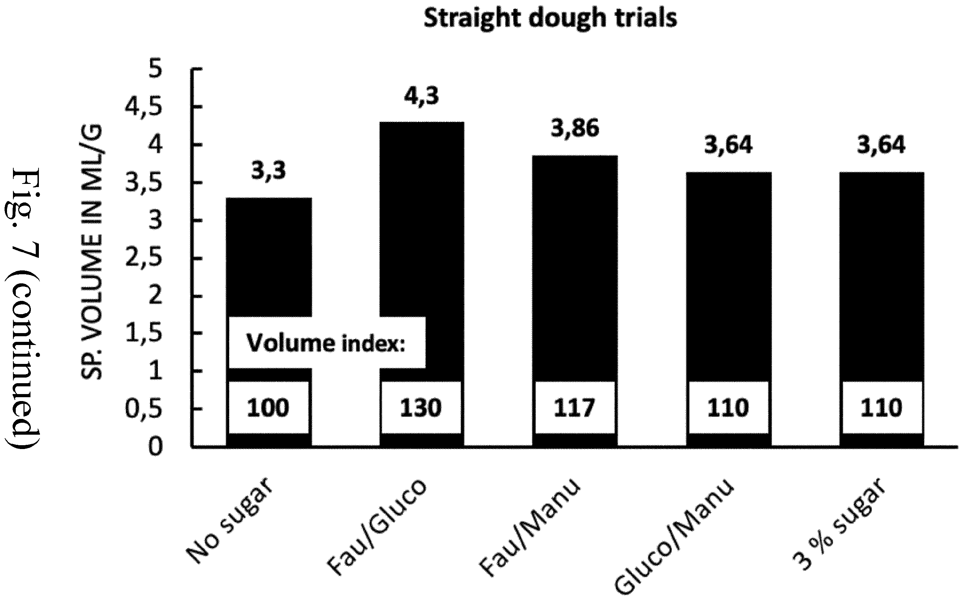

Another observation made by the inventors is that it seems as if the presence of the combination of amyloglucosidase and maltogenic amylase also have impact on this first enzymatic step, as the release of sugars seems to be faster when all three enzymes are present compared with the presence of alpha-amylase alone. Thus, in this first step, both amyloglucosidase and maltogenic amylase may contribute to the release of sugar, even if they have a lower relative activity at the proofing temperature compared with the relative activity at higher temperatures (eg at about 60 °C). As seen from Figure 7 all three enzymes (thermolabile amylase, thermostable amyloglucosidase and maltogenic amylase) contribute to the proofing of the dough. This observation supports the hypothesis by the inventor that even if the amyloglucosidase used is thermostable, it has some activity at proofing temperature, which normally is in a range of from about 20 to about 40 °C. The same applies to the maltogenic amylase.

An envisioned scenario could be that the maltogenic amylase contributes to the release of maltose from starch and that some of the maltose released from the starch by action of alpha-amylase and (maybe) by action of maltogenic amylase is further degraded to glucose by action of the amyloglucosidase present in the combination.

Another challenge in restraining from adding sugar to the dough is how to obtain a baked product that is palatable. To this end, taste, smell, aroma, consistency etc. of the baked product influence whether a consumer finds the baked product palatable. As seen from the examples herein, it has been possible to obtain palatable baked products without adding of sugar to the dough. As demonstrated in the examples herein, the process of the invention leads to baked products with a low content of mono- and di-saccharides. Thus, even if the sugar content is low, the baked product is palatable. The total concentration of mono- and di-saccharides (measured as fructose, glucose, lactose, maltose and saccharose) is at the most about 10% w/w of the baked product, and the concentration of the individual sugar is:
Fructose: at the most 1% w/w, notable 0.7% w/w or less,
Glucose: at the most 4.5 % w/w, notably 4.1 or less,
Lactose: at the most 0.1% w/w, notably not detectable,
Maltose: at the most 5.5% w/w, notably 5.2% or less,
Saccharose: at the most 0.1% w/w, notably not detectable,

The concentration of individual sugar is based on the total weight of the baked product.

The present invention also relates to baked product with the above-mentioned contents of sugars. Baked products with a low content of sugars are highly relevant for many consumers. According to a recent survey 60% of consumers both in Europe and in the USA reported that they are trying to reduce their sugar consumption.

### Cocktail of three enzymes

As mentioned above, a cocktail of three enzymes is used in a process of the present invention. In the following is given details regarding the enzymes including the activity of the enzymes. However, it is to be borne in mind that the activities stated are measured in *in vitro* systems under standardized conditions (temperature, pH, humidity etc.). The activity of an enzyme is dependent on the conditions under which the enzyme is present. Thus, the activity in a dough and during baking may differ from the *in vitro* activities given herein.

The *alpha-amylase* used (EC 3.2.1.1) is an enzyme, which hydrolyzes the degradation of alpha-1,4-glucosidic bonds in oligo- or polysaccharides such as in starch to yield maltose, but it does not act on maltose itself. Intermediate oligosaccharides such as dextrins are formed in the process. Alpha-amylase is an endoglucosidase which cleaves an internal glucosidic bond within an oligo- or polysaccharide.

The alpha-amylase may be an alpha-amylase of fungal or bacterial origin. Preferred is an alpha-amylase of fungal origin. The fungal origin may be from Aspergillus, such as *Aspergillus oryzae, Aspergillus niger* or *Aspergillus kawachii.* Examples of commercially available compositions comprising alpha-amylases are FUNGAMYL^{™}, including Fungamyl 4000 SG and Fungamyl Prime BAN^{®} (all from Novozymes, Denmark), MYCOLASE^{®}, Bakezyme P180, Bakezyme P500 (DSM, Gist Brocades), Grindamyl A 1000, Grindamyl A 5000, Grindamyl A 10000, Grindamyl A 14000 (from IFF/Dupont) and Veron M4 /from AB Enzymes). Alpha-amylases of bacterial origin suitable for use in the present invention includes Biobake 2500 (Kerry Ingredients), BAN 800 MG (Novozymes), Bakezymes AN 301 (DSM) and Grindamyl Max life (IFF/Dupont.

Especially suitable for use in the present invention is an alpha-amylase, which is a fungal alpha-amylase that is an endo-amylase that hydrolyzes (1,4)-alpha-D-glucosidic linkages in starch polysaccharides, and the fungal alpha-amylase is obtained from Aspergillus oryzae.

Alpha-amylases are normally used in bread inter alia to improve brown curst colour, to ensure fine and uniform crumb structure and/or to increase the volume of the bread.

However, in the present invention, an important feature of the alpha-amylase is its ability to degrade starch to provide mono- and disaccharides for use in the yeast fermentation process.

As it appears from the examples herein, a suitable alpha-amylase for use in the present invention is contained in the commercial product Fungamyl^{®} 4000 SG from Novozymes, Copenhagen. Other alpha-amylases have also been tested and found suitable for use. These include the enzymes Fungamyl from Novozymes, Grindamyl from IFF/Dupont, Bakezyme from DSM and Veron M4 from AB Enzymes.

Fungamyl^{®} 4000 SG contains alpha-amylase from *Aspergillus oryzae.* It has an activity of 4000 FAU-F/g. According to a datasheet from Novozymes, the enzyme is yellow to light brown and appears as a granulate having a particle size of approx. 50-212 microns; it has an approx. density of 0.6 g/ml.; it is readily soluble in water at all concentrations that occur in normal usage. Other alpha-amylases having the same characteristics, or characteristics that deviate at the most 10% from the characteristics mentioned above are contemplated to be suitable for use in the present invention; fx: one characteristic is the activity - this may be within a range of from 3600 - 4400 FAU-F/g; the particle size may be from 45 - 233 micron and the density may be from 0.54-0.66 g/ml. FAU-F is a measure for enzyme activity. FAU refers to Fungal Alpha-amylase Unit, ie the amount of enzyme which breaks down 5.26 g starch per hour at Novozymes' standard method for determination of alpha-amylase. Tests for alpha-amylase activity are well known in the art. See for example test described on Sigma Aldrich, or in Bernfeld, P. (1955) Methods in Enzymology 1, 149-158. Further methods are published in Enzymology, and the selection and application of methods are within the skill of a person skilled in the art.

The alpha-amylase has an activity in a range of from 30 to 65 °C (relative activity at least 40% of maximal activity) and about 100% relative activity at a temperature of 50 to 55°C. Likewise, it has an optimum activity at a pH in a range of from 3.5 to 7.3 (more than 30% relative activity) and about 100% relative activity at about pH 4.5 to about 5.3. At temperatures from approx. 60-65 °C, the activity decreases and at approx. 75°C, the enzymes is 100% inactivated. The alpha-amylase begins rapidly to be inactivated at temperatures greater than about 55 °C, i.e. before the starch gelatinizes (at about 60°C) and is believed to have no or only little initial activity during the baking at about 60 to about 100 °C. Therefore, the alpha-amylase is believed to have no (or only little) contribution to the final sugar content in the baked product.

The commercial product, Fungamyl^{®} 4000 SG, contains approximately 59% w/w alpha-amylase CAS No. 900-90-2 (defined as enzyme concentration on dry matter basis), approximately 14% w/w wheat flour CAS No. 130498-22-5, approximately 10% w/w wheat starch CAS No. 9005-25-8, approx. 10% w/w of water CAS No. 7732-18-15 and approximately 7% w/w of dextrin CAS No. 9004-53-9.

When Fungamyl^{®} 4000 SG is employed, it is normally used in an amount in a range for from about 5 to about 15 ppm/kg flour such as in a range of from about 6 to about 12 ppm/kg flour or in a range of from about 7 to about 10 ppm/kg flour such as about 8 ppm/kg flour. If another alpha-amylase is used a person skilled in the art will know how to calculate a suitable amount based on the activities given for Fungamyl^{®} 4000 SG and the other alpha-amylase used.

As used herein for alpha-amylase such as Fungamyl^{®} the following applies:
5 ppm ≈ 20 Fau
7 ppm ~ 28 Fau
10 ppm ≈ 40 Fau
12 ppm ≈ 48 Fau
15 ppm ≈ 60 Fau

A thermolabile alpha-amylase is typically used in an amount corresponding to a range of from about 20 to about 48 Fau/kg flour such as from about 28 to about 40 Fau/kg flour.

Other commercial products may contain the same alpha-amylase or another alpha-amylase suitable for use in the present invention. Such products are also contemplated to be suitable for use in the present invention.

Compared with the Fungamyl^{®} 4000SG product, a suitable alpha-amylase composition may be a composition, wherein there may be a variation in the content of flour (another flour than wheat may be used), there may be a variation in the concentration of flour (another concentration than 90% by weight may be used) etc. Thus, a composition comprising an alpha-amylase suitable for use in the present invention may comprise:
Alpha-amylase in a concentration range of from 50 to 70% w/w.
Flour in a concentration range of from 10 to 25% w/w.
Starch in a concentration range of from 5 to 15% w/w,
Water in a concentration range of from 0.5 -2% w/w.
Dextrin in a concentration range of from 4 to 12% w/w.

The above-given compositions are only examples of suitable compositions. Other compositions may also be suitable provided that they contain an alpha-amylase suitable for the present use. In general, such compositions contain one or more ingredients that make the enzyme stable for storages or that enable easy handling of the enzymes. A suitable composition may be in solid form or it may be in the form of a liquid.

Minor amounts of trace elements from the production process may be present in a composition. Normally, not more than a few percent at maximum is present.

*Glucoamylase* (1,4-alpha-D-glucan glucohydrolase, EC 3.2.1.3), also denoted amyloglucosidase, is an enzyme, which catalyses the release of beta-D-glucose from the non-reducing ends of starch or related oligo- and polysaccharides.

The glucose sweetness intensity obtained in the final baked product is believed to come mainly from the action of the amyloglucosidase and its ability to release glucose. Moreover, it is believed to participate in the Maillard reaction yielding a richer, golden crust of the baked product.

An amyloglucosidase for use in the enzymatic combination for use in the present invention is an enzyme, which has optimum activity at about 60-65 °C and which has almost no activity at temperatures exceeding 75 °C. It has about 50% relative activity at a temperature of from about 40 to about 75 °C, the activity being measured at pH 5.0 after 30 min incubation time at the relevant temperature.

Amyloglucosidases are normally used in the baking industry to obtain more colour.

As it appears from the examples herein, a suitable amyloglucosidase is GoldCrust 3300BG from Novozymes, Denmark. Other suitable amyloglucosidases are Grindamyl AG 1500C, FD48, plussweet G (all from IFF/Dupont), Bakezyme AG 800 and Bakezyme AG 1100 (both from DSM) and AMG 1100 BG (from Novozymes).

Gold Crust 3300BG is derived from *Aspergillus niger.* The activity of the enzyme is expressed in Amyloglucosidase Units/g (AGU/g measured under reaction conditions pH = 4.3, temperature 37 °C and an incubation time of 6 min. The enzyme activity is determined based on the release of glucose and calculated relative to an enzyme standard (ESFA Journal, 16 (10), October 2018 - https://dol.org/10.2903/j.efsa.2018.5450.

When Gold Crustl^{®} 3300BG is employed, it is normally used in an amount in a range for from about 100 to about 500 ppm/kg flour such as in a range of from about 150 to about 400 ppm/kg flour or in a range of from about 175 to about 400 ppm/kg flour such as about 200 or 400 ppm/kg flour. If another amyloglucosidase is used a person skilled in the art will know how to calculate a suitable amount based on the activities given for Gold Crust^{®} 3300BG and the other amyloglucosidase used.

As used herein for a thermo-stable amyloglucosidase such as Gold Crustl^{®} the following applies:
150 ppm ≈495 AGU
175 ppm ≈ 578 AGU
200 ppm≈ 660 AGU
400 ppm ≈ 1320 AGU
500 ppm ≈ 1650 AGU

A thermostable amyloglucosidade is typically used in an amount corresponding to a range of from about 578 to about1650 AGU/kg flour such as from about 660 to about 1650 AGU/kg flour.

The above-given composition is only an example of a suitable composition. Other compositions may also be suitable provided that they contain an alpha-amylase suitable for the present use. In general, such compositions contain one or more ingredients that make the enzyme stable for storages or that enable easy handling of the enzymes. A suitable composition may be in solid or liquid form.

A *maltogenic amylase* (EC 3.2.1.133) is able to hydrolyze starch, amylose and amylopectin to maltose. A maltogenic amylase may be produced from bacteria such as *Bacillus subtilis* (Novamyl^{®} 10000 BG) or *Bacillus stearothermophilus.* The enzyme used in the Examples herein is from *Bacillus subtilis.*

Maltogenic amylases are normally used in the baking industry for improving softness.

The crust of bread forms through the Maillard reaction, which is a chemical reaction between sugars and amino acids that occur at high heat. Amino acids are abundant in the flour and are not a limiting factor, whereas the content of sugar is believed to be a limiting factor.

As demonstrated in the examples herein, the maltogenic amylase contained in the commercial product Novamyl^{®} 10000 BG has proved to be suitable in the present context. Other suitable maltogenic amylases are Grindamyl Max life P100, U4, E50, Powerfresh 8100, Powerfresh 3000, Powerfresh 9740, Powerfresh 9450, Powerfresh 9460, Powerfresh 7001, Powerfresh 7002 (all from IFF/Dupont), Novamyl 3D, Sensea BG, Novamyl Rye, Novamyl Pro 80 BG and Novamyl Ro 12 BG (all from Novozymes), Bakemaster Master, Bakemaster Fresh XL, Bakemaster Man 10000, Bakemaster Alpha (all from DSM), Veron 1000, Veron AC, Veron BA, Veron Sort+, Veron ELS and Amylofresh (all from AB Enzymes).

Novamyl^{®} 10000 BG contains a maltogenic amylase obtained from *Bacillus subtilis.* It has an activity of 10000 MANU/g. It appears as a light brown powder in the form of a free-flowing, low-dusting granulate having a mean particle size of approximately 50-212 microns. It has an approximate density of 0.6% g/ml. It is readily soluble in water at all concentrations that occur in normal usage. Other maltogenic amylases having the same characteristics, or characteristics that deviate at the most 10% from the characteristics mentioned above, are contemplated to be suitable for use in the present invention; e.g.: one characteristic is the activity - this may be within a range of from 9000 - 11000 MANU/g; the particle size may be from 45 -233 micron and the density may be from 0.54-0.66 g/ml.

MANU is Maltogenic Amylase Novo Units. One MANU is defined as the amount of enzymes that produces 1 µmol glucose per minute using maltotriose as substrate under reaction conditions: pH = 5.0, temperature = 37 °C, incubation time = 30 min. The enzymatic hydrolysis of maltotriose results in the release of glucose, which can be determined quantitatively using a hexokinase assay (EFSA Journal, 16 (5), May 2018 - https://doi.org/10.2903/j.efsa.2018.5171).

The maltogenic amylase has an optimal activity in a range of from 50 to 75 °C (relative activity at least 80%) and about 100% relative activity at a temperature of 57 to 65°C and a pH of 5.5. Likewise, it has an optimum activity at a pH in a range of from 3.5 to 7.0 (more than 30% relative activity) and about 100% relative activity at about pH 4.0 to about 5.0. The effect of temperature on maltogenic amylase activity. Maltogenic amylase was incubated at different temperatures for 30 minutes at pH 5.5 using maltotriose as substrate.

The commercial product, Novamyl^{®} 10000 BG, contains approximately 90% by weight of wheat flour, CAS No. 130498-22-5 5% by weight of sodium chloride CAS NI. 7647-14-5, 4% by weight of maltogenic amylase (defined as enzyme concentration on dry matter basis) CAS No. 160611-47-2 and 1% by weight of water CAS No. 7732-18-5. For more details, reference is given to the data sheet issued by Novozymes and valid from 2017-08-24. Other commercial products may contain the same maltogenic amylase or another maltogenic amylase suitable for use in the present invention. Such products are also contemplated to be suitable for use in the present invention. Compared with Novamyl^{®} 10000 BG, a suitable maltogenic amylase composition may be a composition, wherein there is a variation in the content of flour (another flour than wheat may be used), there may also be a variation in the concentration of flour (another concentration than 90% by weight may be used) etc. Thus, a composition comprising a maltogenic amylase suitable for use in the present invention may comprise:
Flour in a concentration range of from 80 to 95% w/w,
NaCl in a concentration range of from 2 to 10% w/w
Maltogenic amylase in a concentration range of from 2 to 10% w/w
Water in a concentration range of from 0.5 -2% w/w.

Minor amounts of trace elements from the production process may be present in a composition.

When Novamyl^{®} 10000 BG is employed, it is normally used in an amount in a range for from about 50 to about 300 ppm/kg flour such as in a range of from about 100 to about 250 ppm/kg flour or in a range of from about 125 to about 200 ppm/kg flour such as about 150 ppm/kg flour. If another maltogenic amylase is used a person skilled in the art will know how to calculate a suitable amount based on the activities given for Novamyl^{®} 10000 BG and the other maltogenic amylase used.

As used herein for a maltogenic amylase such as Novamyl -stable amyloglucosidase such as Gold Crustl^{®} the following applies:
50 ppm ≈ 500 Manu
100 ppm ≈1000 Manu
125 ppm ≈ 1250 Manu
150 ppm ≈ 1500 Manu
250 ppm ≈ 2500 Manu
300 ppm ≈3000 Manu
400 ppm ≈4000 Manu

A maltogenic amylase is typically used in an amount corresponding to a range of from about 500 to about 2500 Manu/kg flour such as from about 1000 to about 2500 Manu/kg flour.

The above-given compositions are only examples of suitable compositions. Other compositions may also be suitable provided that they contain an alpha-amylase suitable for the present use. In general, such compositions contain one or more ingredients that make the enzyme stable for storages or that enable easy handling of the enzymes. A suitable composition may be in solid form or it may be in the form of a liquid.

The enzymes also impart other beneficial properties to the final product. Thus, as seen from the examples herein, the final product has excellent properties with respect to crust colour, shape of the product, uniformity, cell size, cell wall, cell form and crumb colour.

The dough may also contain other enzymes such as thermo-stable alpha-amylases, lipases, xylanases etc. However, these enzymes do not contribute to the content of sugar in the final product nor to the proofing time observed.

### Ingredients in the dough

The flour used in the present process can be of any origin, provided it contains the necessary amount of damaged starch. The flour may be wheat flour, whole wheat flour, heat-treated flour, cake flour, rye flour, sifted rye, oat flour, barley flour, triticale (bread) flour, rice flour, corn flour, potato flour, heat-treated flour, bleached flour, or mixtures thereof, and/or it may include tapioca starch, corn starch, potato starch etc.

As demonstrated in the examples herein, a flour suitable for use in the present invention is wheat flour. The wheat flour may be any suitable wheat flour for example, one or more selected from the group consisting of all-purpose flour, bread flour, German type 550 flour, Reform flour, Manitoba flour, durum wheat flour, wheat flour based on soft or hard wheat types, Emmer, spelt & cake flours, other commercially available wheat flour, and combinations thereof.

There are generally speaking four types for wheat flour. White flour is made from endosperm only of the wheat grain. Brown flour includes some of the grains's germ and bran, while whole grain or wholemade flour is made from the entire grain, including the bran, endosperm and germ. Germ flour is made from the endosperm and germ, excluding the bran. All four types are suitable for use in the present invention.

As mentioned above, the flour used must have a certain content of damaged starch. In the present invention a content of at least 5% w/w (based on the total weight of the flour). Generally, the content of damaged starch is at the most 20% w/w. Thus, a suitable content of damaged starch is in a range of from 5 to 20% w/w or 6% w/w or more, 7% w/w or more, 8% w/w or more, 9% w/w or more, 10% w/w or more, 11% w/w or more or 12% w/w or more. In the examples herein, flour is used, wherein the content of damaged starch is from 5 to 12%, notably from 7 to 9% w/w (based on the total weight of flour) is suitable. If flour is used in combination with a starch, the total content of damaged starch is as described above.

Various starches may also be used as supplement to the flour.

A yeast suitable for use in the present invention is any yeast conventionally used in yeast-raised bakery. Suitable yeasts include dry yeast, sugar stable yeast and normal yeast. An especially suitable yeast is *Saccharomyces cervisae.* In the examples herein Danish standard yeast (Malteser yeast) has been used.

If necessary, pH of the dough is adjusted to a pH in a range of from about 4 to about 5 such as from about 4.5 to 5 by addition of one or more pH-adjusting agents. The adjustment of pH may be made to ensure the desired activity of the enzymes.

The dough may also comprise other commonly used ingredients in a dough. Such ingredients are typically mixed together with the other ingredients during preparation of the dough. Suitable additives include one of more of:
emulsifiers, fibers (such as maltodextrins, polydextrose, inulins, etc.), triglyceride, fat, pH adjusting additives, proofing adjusting additives, shortening agents, dough strengtheners, flour improvers, other enzymes including such enzymes that strengthen the dough; oxidizing enzymes, hemicellulose, lipase, protease, combinations thereof, ascorbic acid, sodium chloride, preservation agent, chemical leaving agent and other commonly used ingredients in baked products.

Mixing can be done with any suitable methods including a continuous mixer system, a spiral mixer of a fork mixer.

After mixing all the ingredients, the dough obtained is left to proofing at suitable conditions. As mentioned above, the proofing time is markedly reduced compared with doughs having added sugar. The proofing time may be reduced by 30% such as 40% or even 50%. The proofing is normally carried out at slightly elevated temperatures compared to room temperature. The temperature is normally in a range of from about 20 to about 40 °C, notably from about 25 to about 35 °C or from about 25 to about 30 °C and at a relative humidity in a range of from 75-90% RH.

The dough may also be obtained using other methods such as sponge dough, straight dough, poolished dough, liquid sponge, CBP (Chorleywood bread process), long fermentation or freezing technology. A sponge dough is a two-step bread making process. In the first step a sponge is made and allowed to ferment for a period of time, and in a second step the sponge is added to the final dough's ingredients. A straight dough is a single-mix process of making bread. The dough is made from all ingredients, and they are placed together and combined in one kneading or mixing session followed by fermentation. The CBP (Chorleywood bread process) process allows the use of lower-protein wheats and reduces processing time. The dough may also be prepared in a stepwise manner. Such a stepwise manner could include a pre-step to soften the dough followed by addition of the enzymes and the proofing the dough. In general enzymes are not added in the pre-step, but there could be situations, where inclusion of one or more of the enzymes is beneficial for the end result of the final product.

The dough obtained after mixing and the dough obtained after proofing are also described herein.

Thus, the dough for use in the process of the invention can be
i) a dough before proofing and which is primarily frozen to delay proofing (non-proofed dough),
ii) a frozen, optionally shaped dough, i.e. a dough after proofing and optionally shaped into the desired form followed by infreezing and stable freezing (pre-proofed dough)
iii) a pre-baked product, i.e. the dough is proofed, shaped into the desired form and 70-85% pre-baked,
iv) a baked product, i.e. the dough is proofed, shaped into the desired form and baked.

The following notations are also used: i) thaw and serve (ready to eat after thawing), ii) thaw and bake (par-baked), iii) pre-proofed dough (freezer to oven), and iv) raw dough (should be proofed and baked)

Thus, bake-off products are also objects of the present invention, ie proofed dough that has been baked to a certain extent, but the product needs further baking before intake thereof (pre-baked products).

The dough of the present invention contains flour with a content of damaged starch in a range of from 5% w/w or more (based on the total content of flour) such as from 5 to 10% w/w, from 6 to 10% w/w or from 7 to 9% w/w, a thermo-labile alpha-amylase and a composition containing a thermo-stable amyloglucosidase and a maltogenic amylase; a yeast; water, and optionally other ingredients common for preparing a dough. The dough contains no added sugar. The content of damaged flour is described above. In some cases, the dough may also contain one or more fibers such as maltodextrin or inulin. The content of the enzymes has been described herein before.

Before or after proofing, the dough may be shaped into the desired form. It may be subject to freezing, which normally involves a pre-freezing step at a temperature of from about -35 to - 45 such as about -38 °C for a time period of from about 10 to about 40 min such as from about 15 to about 30 min followed by stable freezing at about -18 °C. The frozen product may be thawed before baking or directly placed in an oven for baking.

The freezing step is normally carried out when the product is sold as a pre-proofed or non-proofed product, i.e. the consumer smust baked the product themselves. For the non-proofed product, the consumers must both proof and bake the product themselves.

The dough may be frozen so that the consumer only needs to bake the dough in order to obtain the baked product. Thus, the present invention also relates to the dough obtained after proofing and wherein the dough has been frozen. When the frozen dough is baked it results in a baked product as described in the following. Freezing of the dough is typically used for laminated doughs.

The proofed product is baked or otherwise handled to obtain the final product. The baking is typically carried out at a temperature in a range of from about 150 to 280 °C or from about 180 to 250 °C, and the core temperature of the product is from 60 to 100 °C, from 70 to 100 °C, from 90 to 100 °C or from 95 to 100 °C. Instead of baking, the dough may be subjected to steam and hence, the product is obtained as a steambread. The steaming is normally carried out at a temperature of about 100 °C, but the core temperature of the product is the same as if baking has been employed. The dough may also be subject to proofing followed by cooking and baking. Thus, the step of baking may be replaced by a step of steaming.

At the beginning of the baking process, the temperature of the dough is close to room temperature and as the temperature of the dough rises when placed in the oven, the alpha-amylase will become inactive (or much less active) and the yeast will be inactivated. During this process, the combination of the amyloglucosidase and the maltogenic amylase is active. Whereas the first enzymatic step provided sugar as feed for the yeast, this second step provides sugar to the baked product so that the consumer finds it palatable, tasty, having a distinct pleasant flavour and texture. Therefore, all enzymes are not active (or equally active) at the temperatures, where mixing and proofing take place. Not all sugars that can be produced under optimal conditions for all enzymes present are produced during mixing and proofing as - if this was the case - all sugar content or most of it could be used by the yeast, leaving no particular sugar content in the final baked product. Accordingly, the alpha-amylase and the two enzymes in the combination of amyloglucosidase and maltogenic amylase have different activity patterns at different temperatures.

The baking results in a baked product. Such a product has a total content of mono- and di-saccharides that is higher than that in the ingredients making up the dough. Moreover, as seen from the Examples herein, a softer bun and more crispy croissants are obtained.

### Baked product

The invention also relates to a baked product obtained by the process set out in the appended claims comprising
i) fructose in a concentration of about at the most 2.6 % such as at the most 2.0% or at the most 1% by weight (in the bread types exemplified: 0.7, 0.6, 0,6%), such as no more than 0.8%, by weight no more than about 0.7% by weight.
ii) glucose in a concentration of at the most 4.5% by weight, such as at the most 4.3% by weight, at the most 4.2% by weight or at the most 4.1% by weight. Glucose may be present in a concentration range from about 3.5 to about 4,5% by weight (in the bread types exemplified: (3.8, 4.1, 3.7%),
iii) lactose in a concentration of at the most 0.5% by weight, or at the most 0.4% by weight, at the most 0.3% by weight, at the most 0.2% by weight or at the most 0.1% by weight (in all bread types exemplified the concentration was 0.1% or less (not detectable)),
iv) maltose in a concentration of at the most 5.5% by weight, such as at the most 5.4% by weight, at the most 5.3% by weight, or in a concentration range of from 2.5 to 5.5% by weight (in the bread types exemplified: 4.3, 5.2, 3.2),
v) saccharose in a concentration of at the most 0.5% by weight, or at the most 0.4% by weight, at the most 0.3% by weight, at the most 0.2% by weight or at the most 0.1% by weight (in all bread types exemplified: 0.1%, or less (not detectable)), wherein the concentration is based on the total weight of the baked product.

Notably, the total concentration of mono- and di-saccharides (measures as fructose, glucose, lactose, maltose and saccharose) is at the most about 10% w/w such as in a range of from 7.5 to 10% w/w based on the total weight of the baked product.

Typically, the concentrations of the individual sugars are:
Fructose: at the most 1% w/w, notable 0.7% w/w or less, such as 0,4% - 0,6%,
Glucose: at the most 4.5 % w/w, notably 4.1 or less, such as 1,7% to 3,7%,
Lactose: at the most 0.1% w/w, notably not detectable,
Maltose: at the most 5.5% w/w, notably 5.2% or less, such as level 2,9% - 3,6%,
Saccharose: at the most 0.1% w/w, notably not detectable,
and the concentration is based on the total weight of the baked product.

Any combination of the concentrations mentioned for the content of the individual sugars in the above paragraphs apply and is within the scope of the present application. Thus, the baked product may obtain:
0.7% by weight of fructose,
4.5% by weight of glucose,
at the most 0.1% of lactose,
2.9% maltose, and
at the most 0.5% by weight of saccharose.

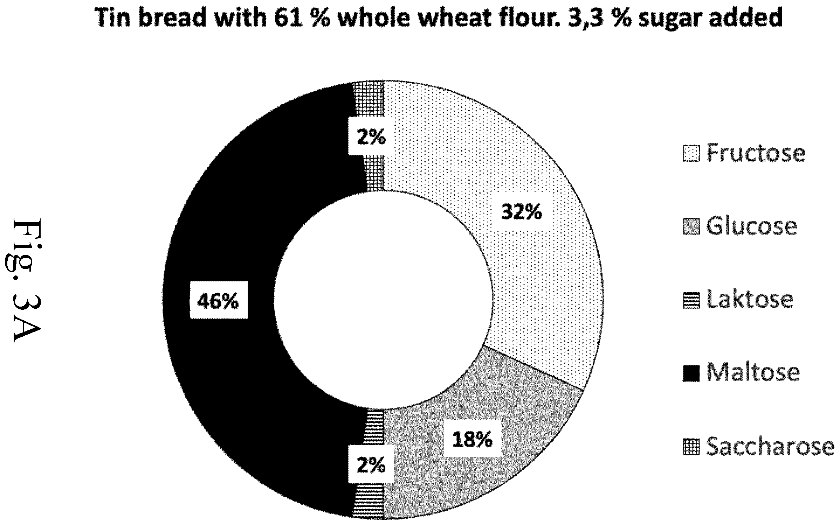

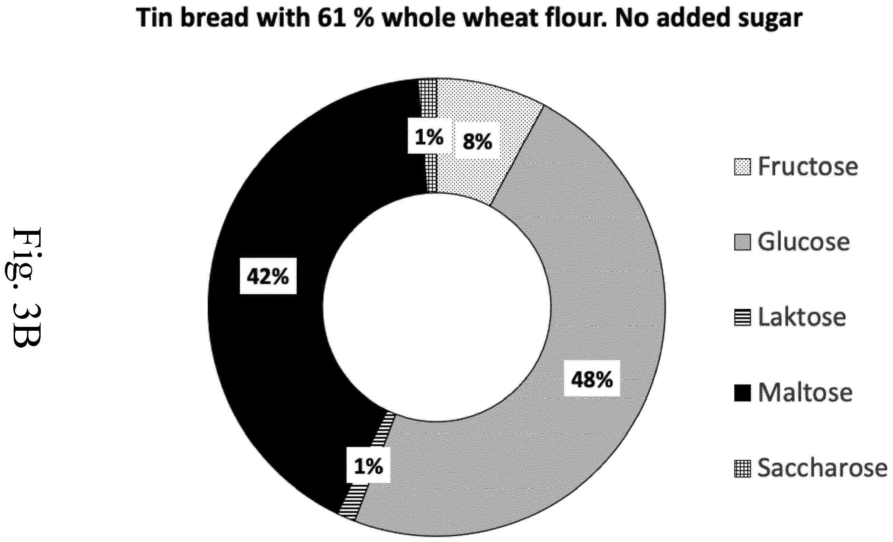

As seen from Figures 3, 4 and 6, the concentration of the individual sugars can also be based on the total amount of sugar in the baked product. Based on the total content of sugars in the product (notably the content of fructose, glucose, lactose, maltose and saccharose), the content of the individual sugars may be:
Fructose: at the most 10% by weight, or at the most 9% by weight, at the most 8% by weight or at the most 7% by weight;
Glucose: at the most 50% by weight, or at the most 49% by weight, at the most 48% by weight, at the most 45% by weight, or in a range of from 25 to 50% by weight, such as at the most about 35% by weight, or at the most about 33% by weight, at the most about 32% by weight, at the most about 31% by weight, or at the most about 30% by weight;
Lactose: at the most about 5% by weight such as at the most about 4% by weight, at the most about 3% by weight, at the most about 2% by weight or at the most about 1% by weight;
Maltose: at the most about 70% by weight such as at the most about 65% by weight, at the most about 60% by weight, or in a range of from about 35 to 70% such as at the most 55% by weight, at the most 50% by weight, at the most 45% by weight, at the most 44% by weight, at the most about 43% by weight or at the most about 42% by weight.

As mentioned herein before, any combination of the content for the individual sugars in the above paragraphs apply and is within the scope of the present application.

A baked product obtained by the processes of the invention may be in the form of burger buns, sandwich bread, whole bread, bread, muffins, pretzels, rolls, tortillas, pizza, bagels, pitas, ciabattas, gluten-free, foccacias, baguettes, loaves, sandwiches, waffles, pan cakes, laminated dough, croissants, pastry puff, cookies and biscuits etc.

When the baking step is steaming, a baked product may be a steambread.

A baked product obtained by a process according the present invention may be consumed by humans suffering from fructose intolerance. Fructose intolerance may be hereditary fructose intolerance (HFI), which is an inborn error of fructose metabolism caused by a deficiency of the enzyme aldolase B. If fructose is ingested, the enzymatic block at aldolase B causes an accumulation of fructose-1-phosphate, which, over time, results in the death of liver cells. Symptoms of HFI include vomiting, convulsions, irritability, hypoglycemia, hemorrhage, and potential kidney failure.

### Synergistic combination of enzymes

The invention also relates to a synergistic combination of a thermolabile alpha-amylase, a thermostable amyloglucosidase and a maltogenic amylase, which combination - when used in a process for producing a baked product without any addition of sugar - results in a baked product that contains 1% w/w or less fructose, notably 0.7% w/w or less, the weight being based on the weight of the baked product. As described herein, it is contemplated that a synergistic effect of the enzymes present in the dough is obtained in the first enzymatic step. Thus, it is envisaged that not only alpha-amylase is responsible for providing sugar to the yeast to consume during fermentation, but there may also be contribution from the combination of amyloglucosidase and maltogenic amylase in such a manner that the maltogenic amylase, although not very active at room and proofing temperature, contributes to the release of maltose, and that the amyloglucosidase contributes to the release of glucose, e.g. from maltose.

Moreover, in those case where it is desired to add an amount of sugar, but in a reduced amount compared to what is normally used, it is possible to replace some of the sugar with the cocktail of enzymes according to the invention. Moreover, replacing an amount of sugar added to a dough by a cocktail of enzymes of the invention results in a dough and a baked product just as good as the product without any reduction in the sugar content. Thus, the cocktail of enzymes of the present invention may also be used in situations where the "no-added sugar" is not the aim, but where a reduction in added sugar is the aim.

The synergistic combination is used in a process according to the invention to obtain a baked product as described above.

The synergistic combination typically comprises the thermo-labile alpha-amylase, the termo-stable amyloglucosidase and the maltogenic amylase in ratios corresponding to
From 20 to 48 Fau or from 28 to 40 Fau of the thermo-labile alpha-amylase,
From 578 to 1650 Agu or from 660 to 1650 Agu of the thermo-stable amyloglucosidase, and
From 500 to 2500 Manu or from 1000 to 2500 Manu of the maltogenic amylase.

Thus, as an example, if the intention is to use 1 g of the synergistic combination/kg flour, then 1 g of the combination should contain from 20 to 48 Fau or from 28 to 40 Fau of the thermo-labile alpha-amylase, from 578 to 1650 Agu or from 660 to 1650 Agu of the thermo-stable amyloglucosidase, and from 500 to 2500 Manu or from 100-2500 Manu of the maltogenic amylase.

Likewise, if the intention is to use 10 g of the synergistic combination/kg flour, then 10 g of the combination should contain from 20 to 48 Fau or from 28 to 40 Fau of the thermo-labile alpha-amylase, from 578 to 1650 Agu or from 660 to 1650 Agu of the thermo-stable amyloglucosidase, and from 500 to 2500 Manu or from 100-2500 Manu of the maltogenic amylase.

The composition of the synergistic combination may be adapted to specific uses. Thus, e.g. for buns or toast, such a synergistic combination may contain the thermo-labile alpha-amylase, the termo-stable amyloglucosidase and the maltogenic amylase in ratios corresponding to
From 24 to 48 Fau or from 24 to 40 Fau of the thermo-labile alpha-amylase,
From 660 to 1650 Agu or from 660 to 1485 Agu of the thermo-stable amyloglucosidase, and
From 500 to 1500 Manu or from 1000 to 1500 Manu of the maltogenic amylase.

### Premix

A bread premix or a premix can be used to obtain a baked product. The premix typically contains flour, the enzyme combinationas described herein, and optionally other ingredients such as emulgators,sodium chloride, yeast, fibers, ascorbic acid, nuts, grains etc.). No added sugar is contained in the premix. The flour may be flour from grains such as wheat flour, corn flour rye flour, barley flour, oat flour, rice flour, sorghum, soy flour, and combinations thereof. The premix may be suitable for obtaining bread, buns etc. or for obtaining products based on laminated dough (such as e.g. croissants). When a product is made from the premix, water and yeast are added to the premix and the dough obtained is ready for proofing and baking.

All details and particulars described herein for one aspect of the invention apply mutatis mutandis to all other aspects of the invention and vice versa.

The following figures and examples are provided below to illustrate the present invention.

They are intended to be illustrative and are not to be construed as limiting in any way.

### BRIEF DESCRIPTION OF THE FIGURES

Figure 1. Figure 1A shows proofing of buns using the same yeast level, but using two doughs, one of which contains 7% w/w sugar (based on the amount of flour used) and the other containing no-added sugar, instead being prepared according to the present invention. The results clearly show a faster proofing of the dough according to the present invention compared to the dough containing added sugar.
Figure 1B shows the impact of different yeast levels on the proofing time. It is seen that almost the same proofing time can be obtained with less yeast using a process of the invention compared to a dough containing 7% w/w added sugar.
Figure 2 shows the appearance of two baked products, one product produced with addition of sugar (marked 1) and the other product according to the invention (marked as 2). Both products have acceptable appearances (see Example 1).
Figures 3A and B show the percent-wise distribution of sugars in tin bread (Example 1); 100% corresponds to the total amount of sugars. Figure 3A shows the results for bread with 3.3% sugar added, and Figure 3B shows the results for bread with no sugar added.
Figures 4A-C show the percent-wise distribution of sugars in wheat bread (Example 2); 100% corresponds to the total amount of sugars. Figure 4A shows the results for wheat bread with 3% added sugar; Figure 4B shows the results for wheat bread with 5% added sugar and Figure 4C shows the results for wheat bread with no sugar added.
Figure 5 shows the appearance of two baked buns. The left-hand product is obtained by addition of 14% sugar (based on the flour content) and the right-hand figure is obtained by a process according to the invention. Both products have acceptable appearances (see Example 3).
Figure 6 shows the percent-wise distribution of sugars in tin bread (Example 3); 100% corresponds to the total amount of sugars.
Figure 7 shows the results of straight dough trials and show a synergistic effect obtained during proofing by use of a thermolabile alpha amylase, a thermostable amyloglucosidase and a maltogenic amylase.
Figure 8A is a baked croissant using 7% sugar in the dough and fig. 8B is a baked croissant according to the invention with no-added sugar.

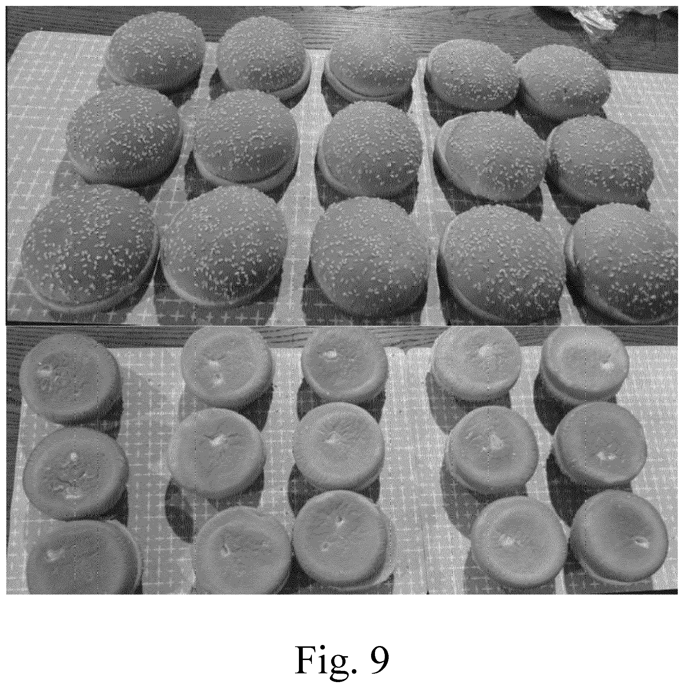

Figure 9 shows the result of Example 8. Test 0 (left), test 4 (right).
Figure 10 shows the height of proofed toast doughs at different time.
Figure 11 shows the pictures of the proofed toast doughs at different time.
Figure 12 shows the height of proofed bun doughs at different time.
Figure 13 shows the pictures of the proofed bun doughs at different time.

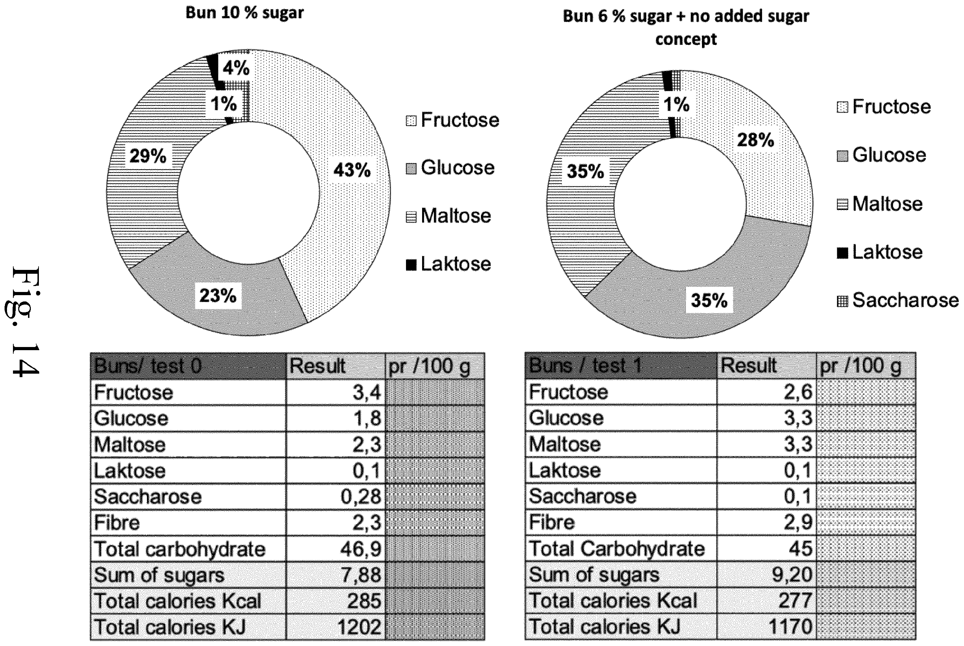

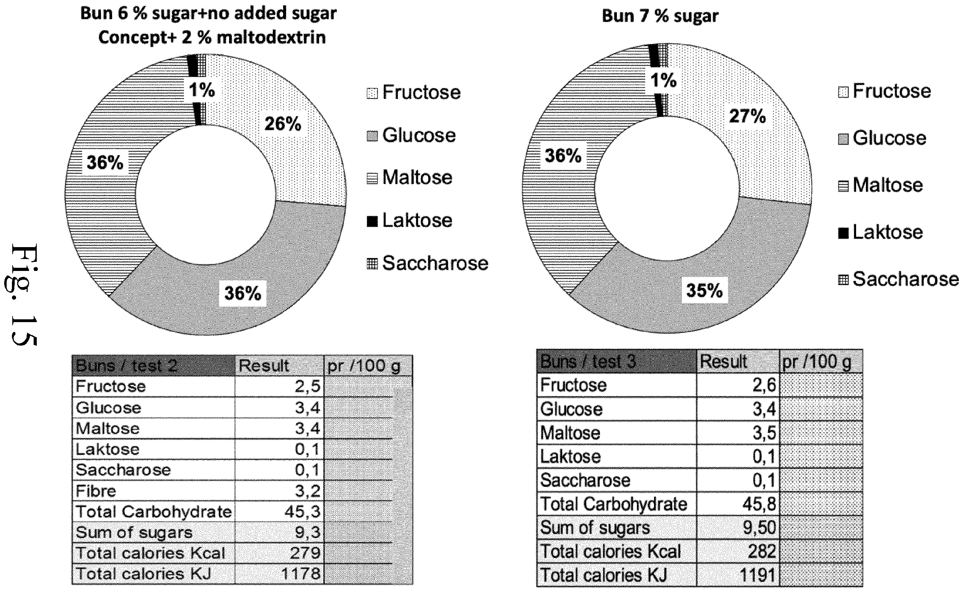

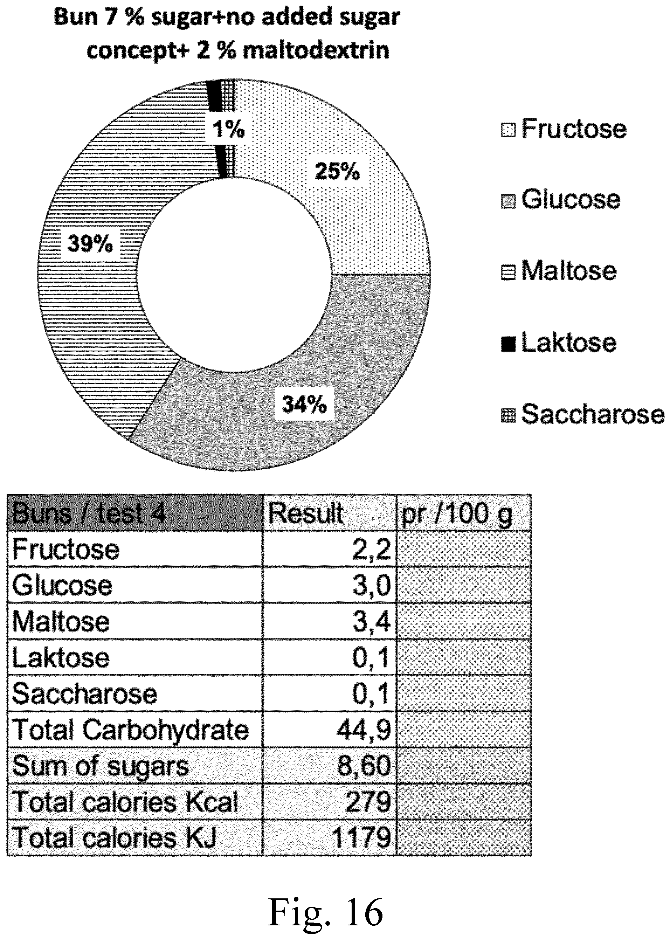

Figures 14-16 show the distribution of individual sugars in buns made with 10% sugar, 6% sugar + combination of enzymes according to the invention, 6% sugar + combination of enzymes according to the invention + 2% maltodextrin, 7% sugar, and 7% sugar + combination of enzymes according to the invention + 2% maltodextrin

### MATERIALS AND METHODS

The analyses of the final baked products, relating to content of individual sugars and calories content were performed by Synlab, Malmo, Sweden. It is an accredited laboratory with No. 1008 and ISO/IEC 17025.

The flour used in the examples all contain damaged starch in a concentration of 7-9% w/w based on the total weight of the flour.

The enzymes: thermolabile alpha-amylase, thermo-stable amyloglucosidase and maltogenic amylase are used in the examples in amounts/kg flour as follows: 8 ppm/kg flour of thermo-labile alpha-amylase, 200 ppm/ka flour or 400 ppm/kg flour of thermo-stable amyloglucosidase and 150 ppm/kg flour of maltogenic amylase. The activity of the enzymes used can be calculated based on the text herein regarding the individual enzymes.

### EXAMPLES

### Example 1 - Preparation of a baked product - whole wheat bread

Two doughs (one without addition of sugar and the other with addition of sugar) were prepared from the following ingredients:

| | C1 (added sugar; comparison) | C2 (no-added sugar) |
|---|---|---|
| | g | g |
| Wheat flour | 800 | 800 |
| White wheat* | 1200 | 1200 |
| Dry sour dough | 20 | 20 |
| Yeast | 90 | 90 |
| Sodium chloride | 30 | 30 |
| Sugar | 66** | 0 |
| Rapeseed oil | 50 | 50 |
| Dark malt | 24 | 24 |
| Improver*** | | 20.0 |
| Improver II**** | 20.0 | |
| Water | 1100 | 1000 |

| | | |
|---|---|---|
| ^{*}Only kernels and flakes from wheat, rye, barley, oats and corn are included in the amount ^{**}about 3.3% sugar in the dough based on the amount of total flour ^{***}contains 8 ppm/kg flour of Fungamyl as thermolabile alpha-amylase, 400 ppm/kg flour of GoldCrust as amyloglucosidase and 150 ppm of Novamyl as maltogenic amylase; and 60 ppm/kg flour of Pentopan 500 (improves structure) **** Standard improver not for sugar release (contains 60 ppm/kg flour of Pentopan 500 BG as xylanase, Fungamyl 800 pmn/kg flour and ascorbic acid 40 ppm/kg flour) | | |

All ingredients were mixed together in a mixer. Mixing a slow speed for 90 sec and at a high speed for 380 sec. A spiral mixer was used. The dough was then proofed for 50 to 55 min at a temperature of 36 °C and a relative humidity of 78%. After proofing the yeast was inactivated at 50 to 55 °C and baked at 180 to 250 °C for 32 min.

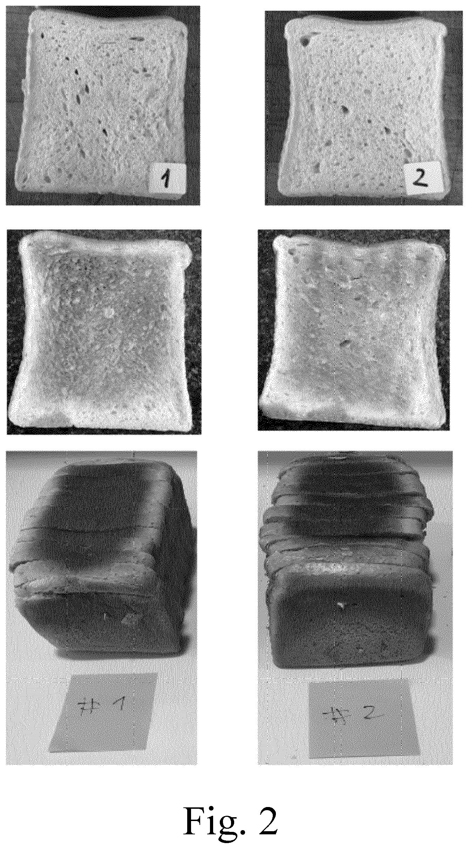

The visual results are shown in Figure 2, where 1 denotes baked product from dough 1.1 and 2 denotes baked product from dough 1.2.

Before baking, the two doughs were evaluated regarding stickiness, softness, extensibility, elasticity and dough temperature. No marked difference was found.

After baking, the bread was evaluated with respect to crust colour, shape of products, uniformity, cell size, cell wall, cell form and crumb colour. No difference was found.

Moreover, the content of individual sugars was evaluated with the following results:

| 3,3% sugar | Result g/100g |
|---|---|
| | |
| Fructose | 1.4 |
| Glucose | 0.8 |
| Laktose | 0.1 |
| Maltose | 2.0 |
| Saccharose | 0.1 |
| Sum of sugar | 4.2 |
| Total calories Kcal | 245 |
| Total calories KJ | 1036 |
| No sugar | Result g/100g |
| | |
| Fructose | 0.6 |
| Glucose | 3.7 |
| Laktose | 0.1 |
| Maltose | 3.2 |
| Saccharose | 0.1 |
| Sum of sugar | 7.5 |
| Total calories Kcal | 236 |
| Total calories KJ | 995 |

Figure 3 shows the content of individual sugars given as a percentage of total amount of sugar.

As seen from the results above, there is a marked change in the content of the individual sugars, especially in the product with no-added sugar compared to the product with added sugar is the reduction low content of fructose, the increase in content of glucose and of maltose are noted. Moreover, in this example a minor reduction in total calories was seen.

### EXAMPLE 2 - Preparation of a baked wheat bread

Three doughs were prepared, one with a content of 3% added sugar (B1), one with a content of 5% added sugar(B2), and one without any added sugar (B3). The sugar content is based on the total amount of flour in the dough. The ingredients were as follows:

| Dough No./Ingredient | | B1 | B2 | B3 | |
|---|---|---|---|---|---|
| | | 3% sugar | 5% sugar | No sugar | |
| Flour | gram | 3000 | 3000 | 3000 | |
| Water | gram | 1620 | 1620 | 1680 | |
| Yeast | Gram | 90 | 90 | 90 | |
| Sugar | Gram | 90 | 150 | | |
| Salt | Gram | 45 | 45 | 45 | |
| Improver I | Gram | 30 | 30 | | |
| Improver II* | Gram | | | 30 | |
| Oil | Gram | 45 | | 45 | |

| | | | | | |
|---|---|---|---|---|---|
| Improver I: Amylase 8 ppm, Xylanase 50 ppm, Lipase 30 ppm (xylanase and lipase impart stability and structure to the final product), Novamyl 150 ppm, Asc 40 ppm; all ppm is ppm/kg of flour; for activity see text herein Improver II:Thermo-stable gluco amylase 400 ppm, thermo-labilemylase 8 ppm, Xylanase 50 ppm, Lipase 30 ppm, Novamyl 150 ppm, Ascorbic acid 40 ppm; all ppm is ppm/kg of flour; for activity see text herein | | | | | |

All ingredients were mixed for 4/6 min in a mixer, the dough is proofed at 36 °C and 78% relative humidity for 50 min, then the yeast was inactivated at 50-55 °C followed by baking of the dough at 180 to 250 °C temperature for 50 min.

After mixing of the ingredients, the doughs were evaluated. All doughs were acceptable. The results were as follows:

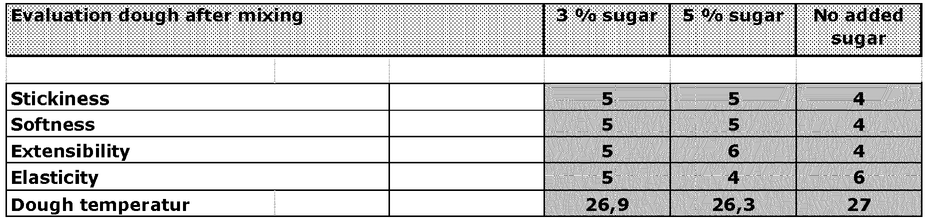

In general, adding of sugar gives a softer dough consistency. Without addition of sugar, the dough becomes less soft and stretchable. However, after baking all products had fine properties with respect to the following parameters.

The evaluation parameters are given below, and they also apply to the other Examples herein

| Stickiness | From 0 to 10 | From little to very | 5 is control |
|---|---|---|---|
| Softness | From 0 to 10 | From less to more | 5 is control |
| Extensibility | From 0 to 10 | From low/short to high/long | 5 is control |
| Elasticity | From 0 to 10 | From low/weak to high/strong | 5 is control |
| Dough temperature | From 0 to 10 | From low to high/strong | 5 is control |

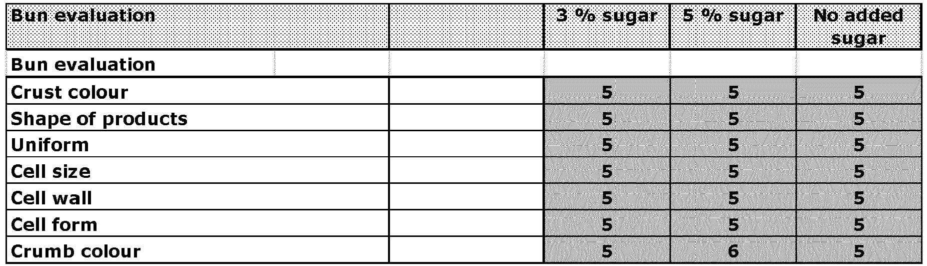

| Crust colour | From 0 to 10 | From light to dark | 5 is control | |
|---|---|---|---|---|
| Shape of product | From 0 to 10 | From low to high | 5 is control | |
| Crumb structure | From 0 to 10 | From less to more | 5 is control | |
| Uniform | From 0 to 10 | From less to more | 5 is control | |
| Cell size | From 0 to 10 | From open to fine/small | 5 is control | |
| Cell wall | From 0 to 10 | From thick to thin | 5 is control | |
| Cell form | From 0 to 10 | From round/deep to elongate/shallow | 5 is control | |
| Crumb colour | From 0 to 10 | From dark to light | 5 is control | |

Regarding taste a lower sugar intensity was noted in the baked product without any added sugar compared to the product, where 5% sugar had been added.

After baking the content of the individual sugars was determined. The following results were obtained - the results for the individual sugars are given as g/100g:

| Sugar | 3% sugar added, B1 | 5% sugar added, B2 | No sugar added, B3 |
|---|---|---|---|
| Fructose | 1.3 | 2.1 | 0.4 |
| Glucose | 0.6 | 1.3 | 1.8 |
| Laktose | 0.1 | 0.1 | 0.1 |
| Maltose | 2.9 | 2.7 | 3.6 |
| Saccharose | 0.1 | 0.1 | 0.1 |
| Sum of sugar | 4.8 | 6.1 | 5.8 |
| Total calories Kcal | 258 | 254 | 249 |
| Total calories KJ | 1094 | 1078 | 1055 |

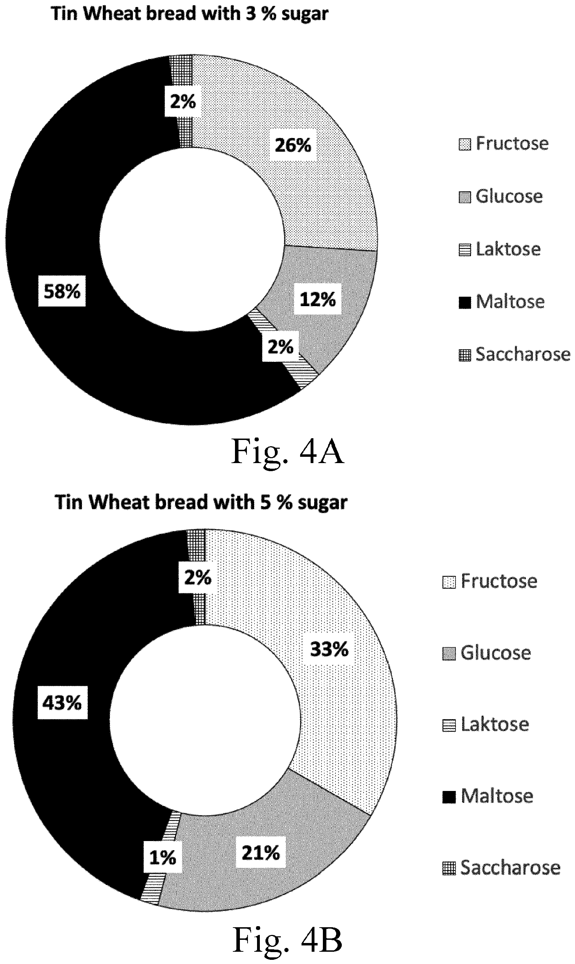

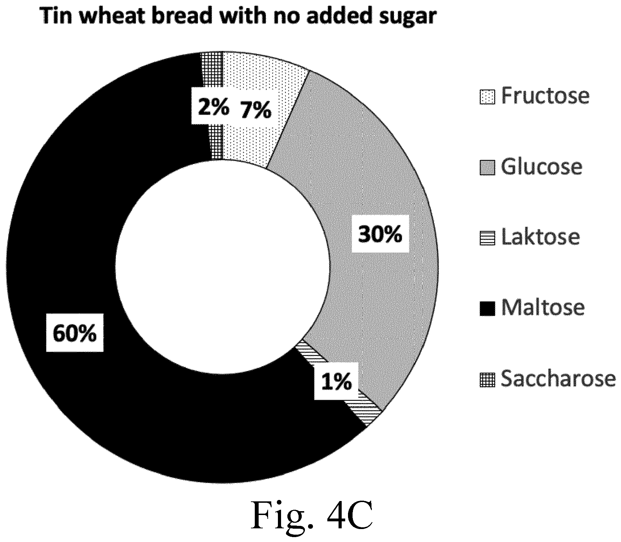

Figure 4 shows the content of individual sugars given as a percentage of total amount of sugar.

### EXAMPLE 3 - Preparation of baked buns

Two types of buns were prepared. One with the addition of 14% sugar (A1) and the other without any addition of sugar. The doughs were prepared with the following ingredients:

| Dough No./Ingredient | | A1 | A2 |
|---|---|---|---|
| | | 14% sugar | No sugar |
| Flour | gram | 2000 | 2000 |
| Water | gram | 1060 | 1220 |
| Yeast | Gram | 110 | 60 |
| Sugar | Gram | 280 | |
| Salt | Gram | 32 | 32 |
| Bun Improver | Gram | 20 | |
| No sugar Improver* | Gram | | 20 |
| Oil | Gram | 100 | 100 |

| | | | |
|---|---|---|---|
| • No sugar Improver comprises alpha-amylase, glucoamylase and maltogenic amylase - corresponds to Improver II in Example 2 • Bun Improver - corresponds to Improver I in Example 2 | | | |

All ingredients were mixed for 60 sec/420 sec in a mixer, the dough is proofed at 38 °C and 84% relative humidity for 50 min, then the yeast was inactivated at 50 C followed by baking of the dough at a temperature of 235/230 °C for 12 min.

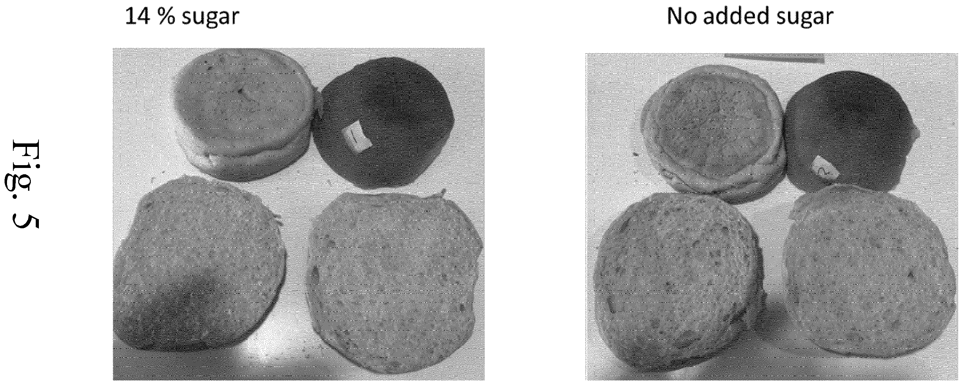

The appearance of the baked buns is illustrated in Figure 5.

After mixing of the ingredients, the doughs were evaluated. All doughs were acceptable. The results were as follows:

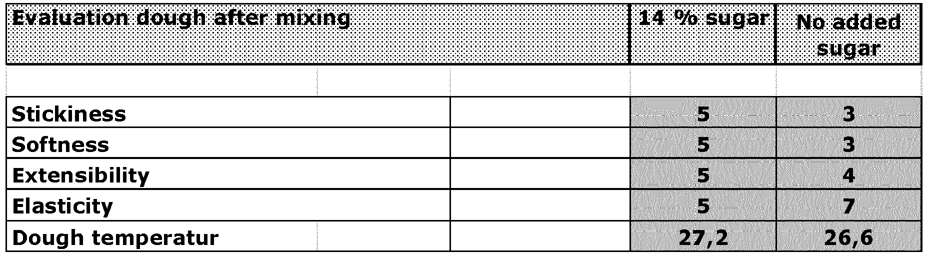

It seems that the dough with added sugar is softer and more stretchable compared to the dough without added sugar. However, these properties do not adversely affect the processing of the dough.

After baking, the evaluation of the buns gave the following results:

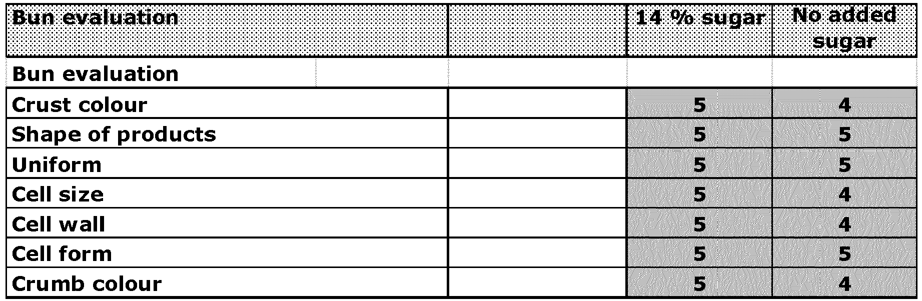

As is seen from the table above, only minor differences were observed. Without addition of sugar, there was a tendency to a more open crumb and a lighter colour. The taste without added sugar is less intense compared to the buns with 14% added sugar.

The content of the individual sugars was measured with the following results:

| 14% sugar | Result g/100g |
|---|---|
| | |
| Fructose | 4.3 |
| Glucose | 3.6 |
| Laktose | 0.1 |
| Maltose | 1.6 |
| Saccharose | 0.1 |
| Sum of sugar | 9.5 |
| Total calories Kcal | 274 |
| Total calories KJ | 1159 |

| No sugar | Result g/100g |
|---|---|
| | |
| Fructose | 0.5 |
| Glucose | 1.7 |
| Laktose | 0.1 |
| Maltose | 2.9 |
| Saccharose | 0.1 |
| Sum of sugar | 5.1 |
| Total calories Kcal | 256 |
| Total calories KJ | 1083 |

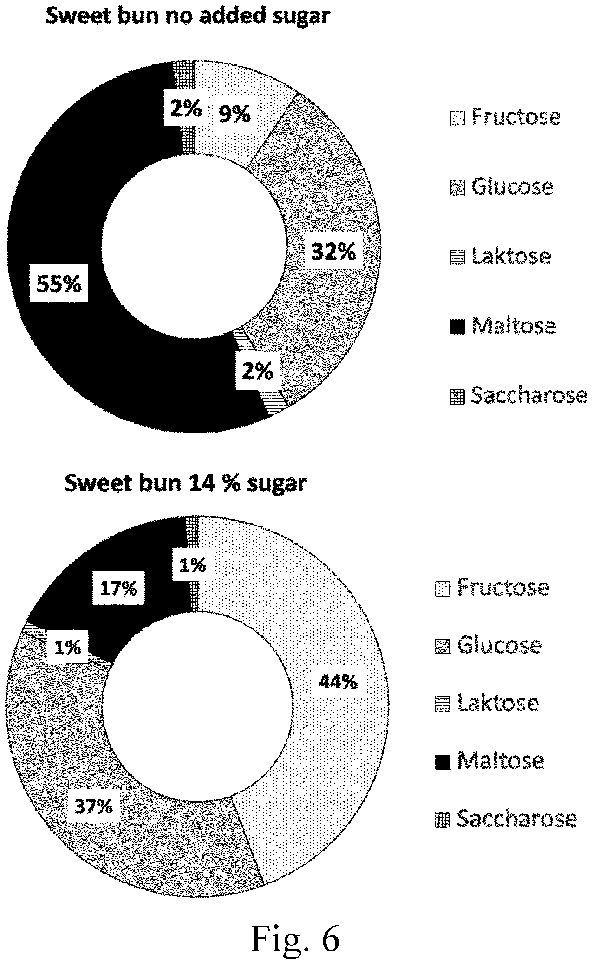

Figure 6 shows the content of individual sugars given as a percentage of total amount of sugar.

As seen from the results above, there is a marked change in the content of the individual sugars, especially in the product with no-added sugar compared to the product with added sugar a reduction low content of fructose, a decrease in the content of glucose and an increase in the content of maltose are noted. Moreover, in this example an approx. 10% reduction in total calories was seen.

### EXAMPLE 4

### Influence of sugar content and yeast content on proofing time

Products based on the following ingredients were prepared

| Dough No./Ingredient | | 1 | 2 | 3 |
|---|---|---|---|---|
| | | 7% sugar | No sugar | No sugar |
| | | 3.5% yeast | 2.5% yeast | 3.5% yeast |
| Flour | gram | 3000 | 3000 | 3000 |
| Water | gram | 1689 | 1689 | 1689 |
| Yeast | Gram | 105 | 75 | 105 |
| Sugar | Gram | 210 | | |
| Salt | Gram | 36 | 36 | 36 |
| Ascorbic acid I | Ppm/kg | 40 | 40 | 40 |
| Oil | Gram | 90 | 90 | 90 |

### Enzymes/additives

| Dough No./Ingredient | | 1 | 2 and 3 |
|---|---|---|---|
| | | 7% sugar | No sugar |
| | | 3.5% yeast | 2.5% yeast or 3.5% yeast |
| Novamyl 10000BG | ppm/kg flour | 150 | 150 |
| | mg/dough | 450 | 450 |
| Gold crust 3300BG | ppm/kg flour | | 350 |
| | mg/dough | | 1050 |
| Fungamyl 4000 SG | ppm/kg flour | 10 | 10 |
| | mg/dough | 30 | 30 |
| Lipoan Etra 1000 | ppm/kg flour | 30 | 30 |
| | mg/dough | 90 | 90 |
| Pentopan 5000 BG | ppm/kg flour | 60 | 60 |
| | mg/dough | 180 | 180 |

The dough and the baked product were made as described in Example 3.

### EXAMPLE 5 - STRAIGHT DOUGH TRIALS

The dough was made from the following ingredient. The thermolabile alpha-amylase, amyloglucosidase and maltogenic amylase tested were added in amounts corresponding to those used in Example 1 or 2.

The enzymes tested were: thermolabile alpha-amylase (Fau), thermostable amyloglucosidase (Gluco), maltogenic amylase (Manu).

### Straight dough recipe for enzyme test:

| Flour | 100 % |
|---|---|
| Water | 57 % |
| Oil (rapeseed) | 2 % |
| Yeast | 3 % |
| Salt | 1,5 |
| Improver I | 1 % |

| Site: | Open pan |
|---|---|
| | 600 g dough |

| Mixing Spiral mixer | Low 60 sec / high 600 sec |
|---|---|
| Floor time | 10 min |
| Scaling dough | 600 g |
| Proofing | To heights/ 50-60 min |
| Baking | 35 min with steam |

The results are shown in Figure 7. The left-hand figure shows a synergistic effect when alpha-amylase and amyloglucosidase is combined and the volume after proofing is increased from 3.3 (no sugar added) to 4.3 (i.e. 30%) or from 3.64 (when 3% sugar was added to the recipe) to 4.3 (i.e. 18%). The volume index, when dough with no enzymes and no sugar is 100, is as follows:
Fau/Glu: 130 g/ml
Fau/Manu: 117 g/ml
Gluco/manu: 110 g/ml
3% sugar: 110 g/ml

The right hand figure shows that addition of alpha-amylase or amyloglucosidase as single enzymes gives increased volume after proofing compared to dough with no enzymes added and either having no sugar added or 3% sugar added. When all three enzymes are added, the best result regarding volume is achieved. The volume index, when dough with no enzymes and no sugar is 100, is as follows:
Alpha-amylase: 121 g/ml
Maltogenic amylase: 97 g/ml
Glucoamylase: 121 g/ml
All three enzymes: 135 g/ml
3% sugar: 113 g/ml

### Dough evaluating

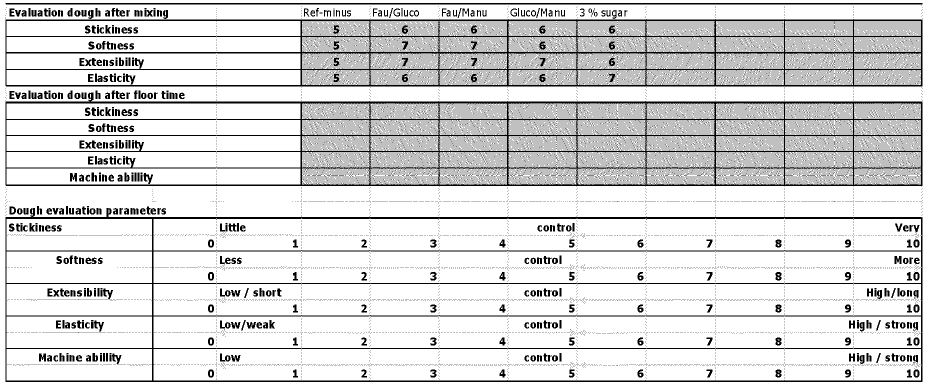

As seen from the table above, the doughs containing enzymes are better than the dough without enzymes and sugar and better or alike the dough containing 3% sugar and no enzymes.

### Volume & crumb evaluation

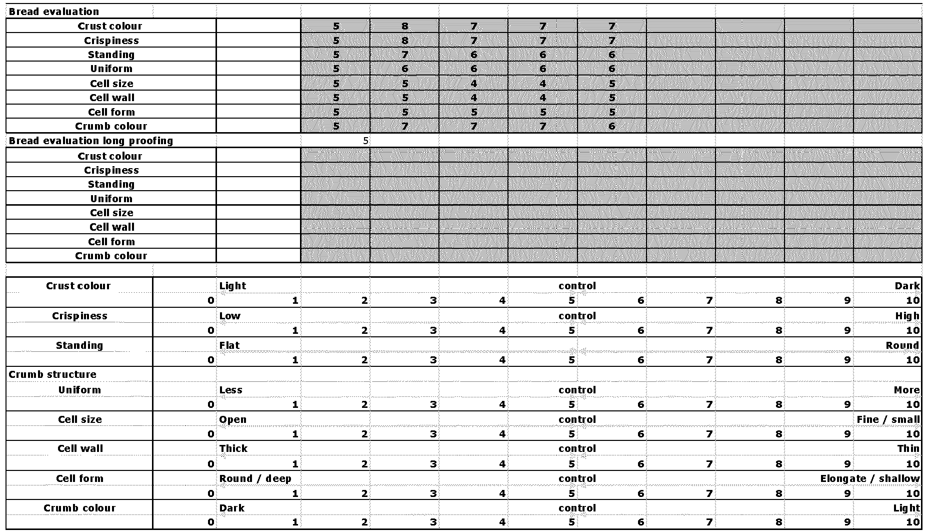

After baking the baked products obtained from doughs containing enzymes are better than the baked product obtained from dough without enzymes and sugar and better or alike the baked product obtained from dough containing 3% sugar and no enzymes. The columns in the table above are the same as in the previous table.

### EXAMPLE 6 - LAMINATED DOUGH - CROISSANTS

Crossaints were made based on the following recipe:

### Crossaint standard recipe:

| Flour | 100 % |
|---|---|
| Water | 50-55 % % |
| Yeast | 8%-10 % |
| Salt | 1,9 % |
| Sugar | 7%-11 % |
| Improver | 0,75 % |
| Butter | 25-35 % |

| Site | 70 g triangle shape | |
|---|---|---|
| spiral mixer | Slow 250 sec | High 290 sec |
| Pre-proofing | 1-3 hours | |
| Proofing | 90 min / 28C/80 rH | |
| Egg wash | | |
| Baking | 18 min/ 180 C | |

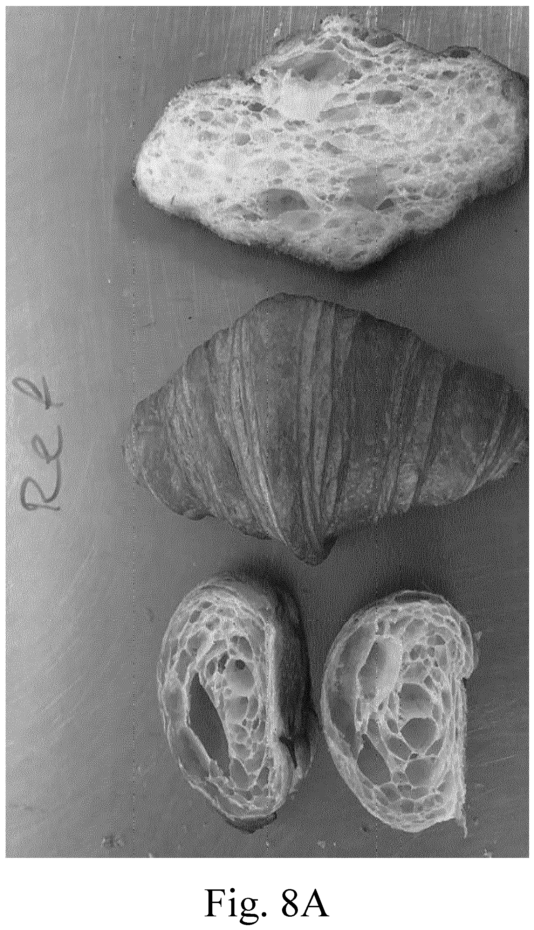

Croissants were made with no-added sugar, but with content of thermolabile alpha-amylase, thermostable amyloglucosidase and maltogenic amylase. The results of the baked croissants are shown in Figure 8A (reference) and 8B (no-added sugar).

Four doughs with no added sugars were made and compared with standard. All baked products resulting from doughs with no sugar added had good as good as or better volume, structure, taste, sugar flavor, color compared with the croissants with 7% sugar content.

In an internal triangle test with 25 participant only one respondent noticed the difference.

### EXAMPLE 7 - Reduction of added sugar

This example illustrate that using the combination of enzymes as claimed herein also can replace 30-40% of added sugar without any lack of quality.

| Ingredient | Test 0 | Test 1 | Test 2 | Test 3 | Test 4 |
|---|---|---|---|---|---|
| Flour kg | 132 | 132 | 132 | 132 | 132 |
| Sugar in % | 10 | 6 | 6 | 7 | 7 |
| Sugar kg | 13.5 | 7.92 | 7.92 | 9.24 | 9.24 |
| 200 ppm GoldCrust= gram | | 26.4 | 26.4 | 26.4 | 26.4 |
| 150 ppm Novamyl 10000 BG=gram | | 19.8 | 19.8 | 19.8 | 19.8 |
| Maltodextrins kg | | | 2.64 | | 2.64 |
| | | | | | |
| Appearance external | Benchmark | | | | |
| Volume | X | Same as benchmark | Lower volume | Same as benchmark | Lower volume |
| Shape | X | Same as benchmark | Same as benchmark | Same as benchmark | Same as benchmark |
| Color | X | Same as benchmark | Same as benchmark | Same as benchmark | Same as benchmark |
| Uneven surface | X | Same as benchmark | Same as benchmark | Same as benchmark | Same as benchmark |
| | | | | | |
| Appearance internal | Benchmark | | | | |
| Resilence | X | Very good | Good | Very good | Good |
| Bide | X | Short bite, but softer in mouth | Short bite, more dry than test 1 | Short bite, but softer in mouth | Short bite, more dry than test 1 |
| Softness | X | Very soft | Softer | Very soft | Softer |
| Uniformity | X | Same as benchmark | Same as benchmark | Same as benchmark | Same as benchmark |
| Crumb structure | X | Same as benchmark | Same as benchmark | Same as benchmark | Same as benchmark |
| Color | X | Same as benchmark | Same as benchmark | Same as benchmark | Same as benchmark |
| Sugar intensity | X | No difference | No difference | No difference | No difference |

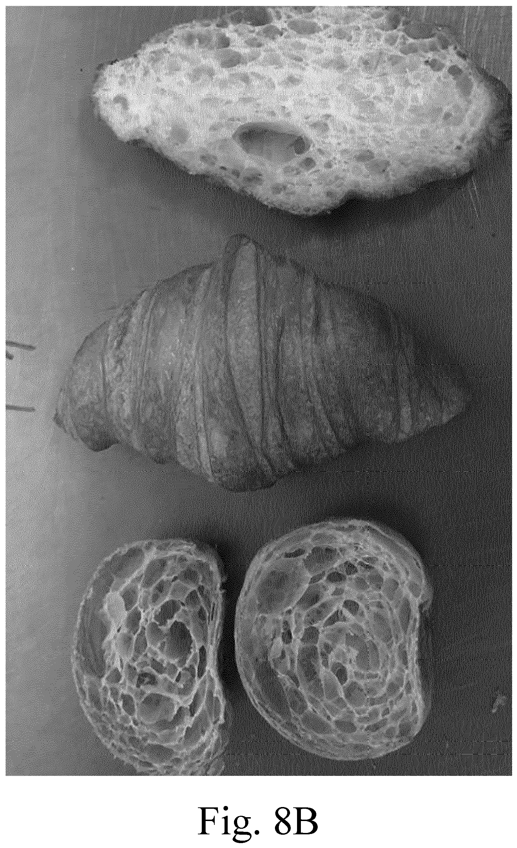

The results show that the combination of enzymes of the invention can replace 30-40% of sugar without any lack of quality, use of the combination of enzymes gives better softness and freshness compared to benchmark and the addition of fiber gives shorter (dry) bide compares to benchmark. Figure 8 shows the result - from left to right: Test 0-Test 4.

### EXAMPLE 8 - Reduction in proofing time

Toast and bun doughs with different content of sugar and with or without the enzyme cocktail of the invention were tested.

### Proofing heights based on toast recipe:

| Proofing heights | 50 g dough ball for proofing | |
|---|---|---|
| Process | Straight dough (toast recipe) | |
| Flour | | |
| | Dana flour from Cerealia | |
| Ingredients | % | *gram* |
| Flour | 100 | 2000 |
| Water | 56 | 1120 |
| Yeast | 3,5 | 70 |
| Sugar | 0%/3 % / 6 % | 0/40 / 100 |
| Oil | 1,5 | 30 |
| salt | 1,8 | 36 |
| Datem | 0,3 | 6 |
| Ascorbic acid | 40 ppm / kg flour | 0,080 |
| No added sugar concept* | 1 | 20 |

- Two doughs with 0% sugar were made; one of which had the three enzymes added ("no-added sugar concept") whereas the other 0% sugar dough did not contain the enzyme blend.Process toast:
   Add all ingredients
   Mix to optimum dough development
   Dividing dough ball into 50 g
   Rounding & molding dough
   Put into cup glass
   Proofing in 30 min / 45 min / 60 min (36C / 84 rH)

The enzymes used were:

### Enzyme solution

| Straight dough | ppm or g | Add dosages pr. kg | Solution you want to add from flour in % |
|---|---|---|---|
| Fungamyl 4000 SG | ppm/kg | 8 | 1 |
| Novamyl 10000 BG | ppm/kg | 150 | 1 |
| GoldCrust 3300 BG | ppm/kg | 400 | 1 |
| Asc | ppm/kg | 40 | 1 |

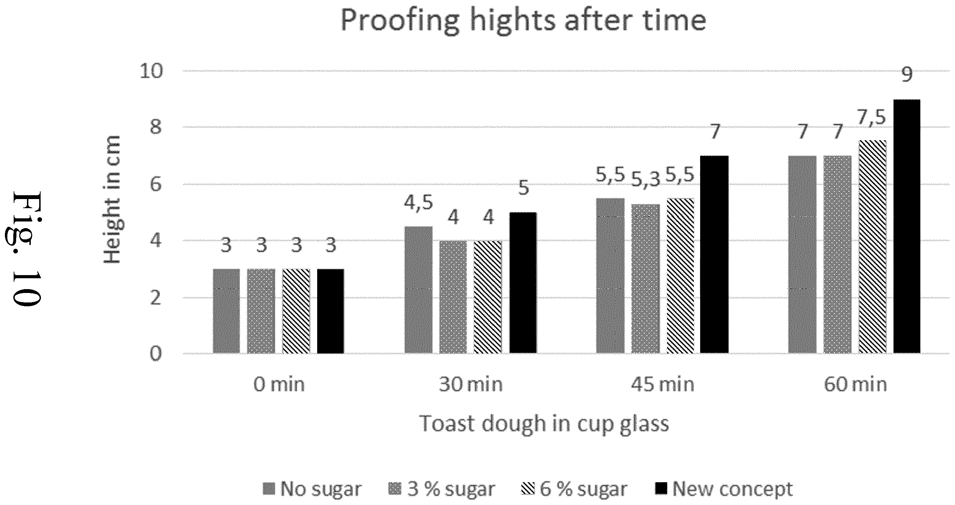

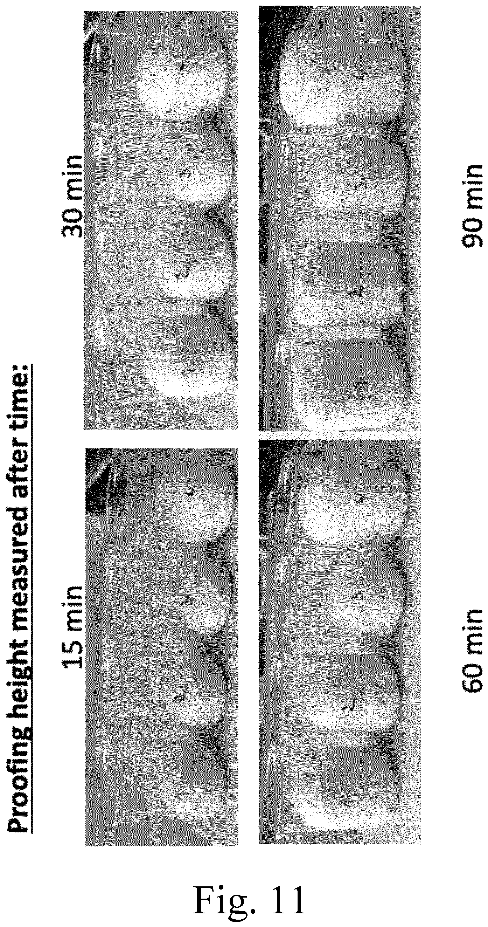

The results are shown in Figs. 10 and 11. As seen from Fig. 10 an increase in height after 30 min is about 66% for the dough with the three enzymes compared with about 33% for the doughs containing 3% or 6% sugar. After 45 min the increase is about 133% for the dough with the three enzymes compared with about 80% for the doughs containing 3% or 6% sugar. After 60 min the increase is about 200% for the dough with the three enzymes compared with about 80-150% for the doughs containing 3% or 6% sugar. In order to gain twice the height compared with the start value, a toast dough containing the combination of the three enzymes accoding to the invention will reach this at least 15 min faster than that obtained for dough with 0%, 3%, 6% sugar without the combination of enzymes.

### Proofing heights based on bun recipe:

| Proofing heights | 50 g dough ball for proofing | |
|---|---|---|
| Process | Straight dough (bun recipe) | |
| Flour | Dana flour from Cerealia | |
| Ingredients | % | *gram* |
| Flour | 100 | 2000 |
| Water | 56 | 1120 |
| Yeast | 4,5 | 90 |
| Sugar | 0% /7 %/ 14 % | 0/140/280 |
| Oil | 5 | 100 |
| salt | 1,8 | 36 |
| Datem | 0,4 | 8 |
| Ascorbic acid | 60 ppm / kg flour | 0,120 |
| No added sugar concept* | 1 | 20 |

- Two doughs with 0% sugar were made; one of which had the three enzymes added ("no-added sugar concept") whereas the other 0% sugar dough did not contain the enzyme blend.

### Process:

Add all ingredients
Mix to optimum dough development
Dividing dough ball into 50 g
Rounding & molding dough
Put into cup glass
Proofing in 30 min / 45 min / 60 min (36C / 84 rH)

The enzymes used were (ppm/kg flour):

### Enzyme solution

| Bun improver | ppm or g | Add dosages pr. kg | Solution you want to add from flour in % |
|---|---|---|---|
| Fungamyl 4000 SG | ppm/kg | 10 | 1 |
| Novamyl 10 000 BG | ppm/kg | 150 | 1 |
| GoldCrust 3300 BG | ppm/kg | 450 | 1 |
| Asc | ppm/kg | 60 | 1 |

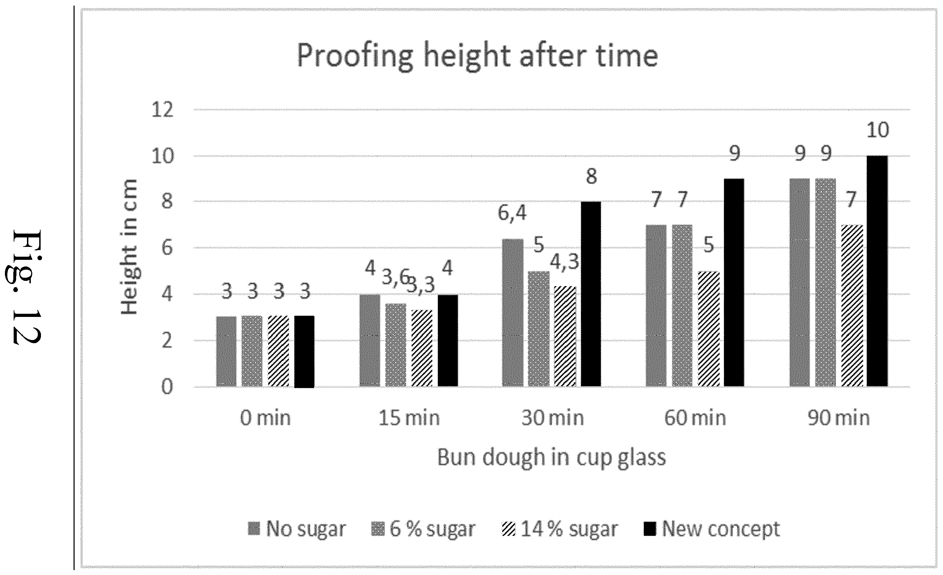

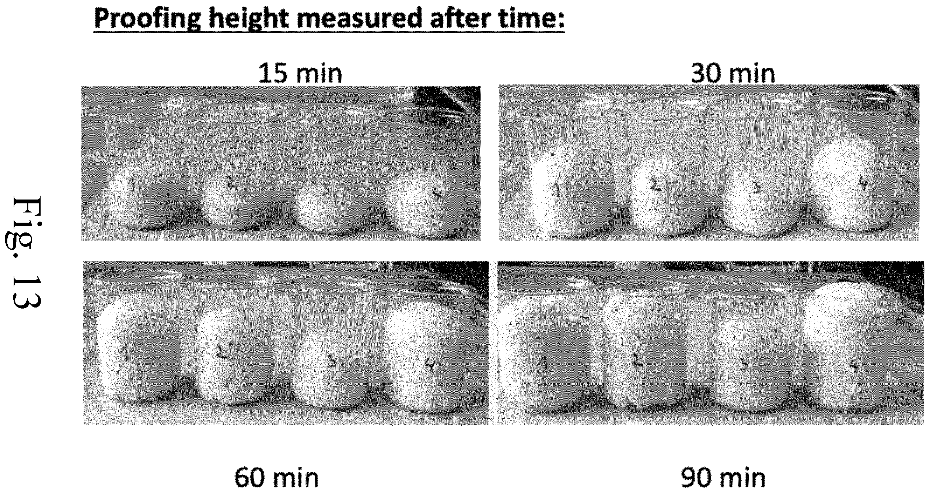

The results are shown in Figs. 12 and 13. As seen from Fig. 12 an increase in height after 30 min is about 266% for the dough with the three enzymes compared with about 43%-66% for the doughs containing 6% or 14% sugar. After 60 min the increase is about 200% for the dough with the three enzymes compared with about 66%-133% for the doughs containing 6% or 14% sugar. After 90 min the increase is about 233% for the dough with the three enzymes compared with about 133%-200% for the doughs containing 6% or 14% sugar. In order to gain twice the height compared with the start value, a bun dough containing the combination of the three enzymes accoding to the invention will reach this at least 15 min faster than that obtained for dough with 6% or 14% sugar without the combination of enzymes.

### EXAMPLE 9 - reduction in sugar content

This example illustrates that it is possible to replace some of the sugar with the enzyme combination according to the invention and obtain products with lower content of fructose and higher content of glucose and maltose. Addition of 2% maltodextrin does not markedly change the content of sugars compared to the buns with the enzyme combination.

The recipe is as follows:

| | Test 0 | Test 1 | Test 2 | Test 3 | Test 4 |
|---|---|---|---|---|---|
| Flour Kg | 132 | 132 | 132 | 132 | 132 |
| Sugar in % | 10% | 6% | 6% | 7% | 7% |
| Sugar in kg | 13,5 | 7,92 | 7,92 | 9,24 | 9,24 |
| 200 ppm Gold crust = Gram | | 26,4 | 26,4 | 26.4 | 26,4 |
| 150 ppm Novamyl 10000 BG = Gram | | 19,8 | 19,8 | 19,8 | 19,8 |
| Malto dextrins (kerry) KG | | | 2,64 | | 2,64 |

The doughs also contain 6-8 ppm thermo-labile alpha amylase (Fungamyl).

## Claims

1. A combination consisting of an alpha-amylase (EC 3.2.1.1), which is thermo-labile and has activity in a range of from 30 to 65 °C, an amyloglucosidase (EC 3.2.1.3), which is thermo-stable and has optimum activity at about 60-65 °C, and a maltogenic amylase (EC 3.2.1.133), which has optimal activity in a range of from 50 to 75 °C, and wherein the thermo-labile alpha-amylase, the thermo-stable amyloglucosidase and the maltogenic amylase are present in ratios corresponding to
from 20 to 48 Fau or from 28 to 40 Fau of the thermo-labile alpha-amylase,
from 578 to 1650 Agu or from 660 to 1650 Agu of the thermo-stable amyloglucosidase, and
from 500 to 2500 Manu or from 1000 to 2500 Manu of the maltogenic amylase.

2. A combination according to claim 1 for use in the preparation of a bakery product with no-added sugar.

3. A process for producing a bakery product with no-added sugar, the process comprising
i) mixing flour with a content of damaged starch of at least 5% by weight with a combination as defined in any one of claims 1-2; a yeast; water, and optionally other ingredients common for preparing a dough, to obtain a dough,
ii) proofing the dough,
iii) optionally shaping the dough into a desired form,
iv) optionally, infreezing the un-baked product at a temperature in a range of from -35 °C - -45 °C for a time period of from about 15 min to about 30 min followed by freezing at about -18°C, and
v) optionally pre-baking or baking the optionally shaped dough at a temperature in a range of from 180 to 250°C.

4. A process according to claim 3 comprising
i) mixing flour with a content of damaged starch of at least 5% by weight with a combination as defined in any one of claims 1-3; a yeast; water, and optionally other ingredients common for preparing a dough, to obtain a dough,
ii) proofing the dough,
iii) optionally shaping the dough into a desired form,
iv) optionally, infreezing the un-baked product at a temperature in a range of from -35 °C - -45 °C for a time period of from about 15 min to about 30 min followed by freezing at about -18°C, and
v) pre-baking or baking the optionally shaped dough at a temperature in a range of from 180 to 250°C.

5. A process according to claim 3, comprising
i) mixing flour with a content of damaged starch of at least 5% by weight with a combination as defined in any one of claims 1-2; a yeast; water, and optionally other ingredients common for preparing a dough, to obtain a dough,
ii) proofing the dough,
iii) optionally shaping the dough into a desired form,
iv) optionally, infreezing the un-baked product at a temperature in a range of from -35 °C - -45 °C for a time period of from about 15 min to about 30 min followed by freezing at about -18°C, and
v) pre-baking the optionally shaped dough at a temperature in a range of from 180 to 250°C, followed by
infreezing the pre-baked product at a temperature in a range of from -35 °C - -45 °C for a time period of from about 15 min to about 30 min followed by freezing at about -18 °C.

6. A process according to claim 3 comprising
i) mixing flour with a content of damaged starch of at least 5% by weight with a combination as defined in any one of claims 1-2; a yeast; water, and optionally other ingredients common for preparing a dough, to obtain a dough,
ii) proofing the dough,
iii) shaping the dough into a desired form,
iv) optionally, infreezing the un-baked product at a temperature in a range of from -35 °C - -45 °C for a time period of from about 15 min to about 30 min followed by freezing at about -18°C, and
v) baking the optionally shaped dough at a temperature in a range of from 180 to 250°C.

7. A process according to claim 3 comprising
i) mixing flour with a content of damaged starch of at least 5% by weight with a combination as defined in any one of claims 1-2; a yeast; water, and optionally other ingredients common for preparing a dough, to obtain a dough,
ii) proofing the dough,
iii) optionally shaping the dough into a desired form,
iv) infreezing the un-baked product at a temperature in a range of from -35 °C - -45 °C for a time period of from about 15 min to about 30 min followed by freezing at about -18°C, and
v) optionally pre-baking or baking the optionally shaped dough at a temperature in a range of from 180 to 250°C.

8. A process according to claim 3 comprising
i) mixing flour with a content of damaged starch of at least 5% by weight with a combination as defined in any one of claims 1-2; a yeast; water, and optionally other ingredients common for preparing a dough, to obtain a dough,
ii) proofing the dough,
iii) shaping the dough into a desired form,
iv) infreezing the un-baked product at a temperature in a range of from -35 °C - -45 °C for a time period of from about 15 min to about 30 min followed by freezing at about -18°C, and
v) optionally pre-baking or baking the optionally shaped dough at a temperature in a range of from 180 to 250°C.

9. A process according to claim 3 comprising
a) mixing flour with a content of damaged starch of at least 5% by weight with a combination as defined in any one of claims 1-2; a yeast; water, and optionally other ingredients common for preparing a dough, to obtain a dough,
b) proofing the dough,
c) baking the dough at a temperature in a range of from 180 to 250 °C.

10. A process according to any one of claims 3-9, wherein the time of proofing to obtain a specified height of the dough is reduced with from 30% to 50% compared with the time of proofing of a dough to the same specified height, wherein the comparison dough has the same ingredients apart from omission of the combination as defined in any one of claims 1-3 and apart from inclusion of 6-7% sugar.

11. A process according to any one of claims 3 -10, wherein the time of proofing to obtain twice the height compared with the start value is at least 15 min faster such as about 15 to 30 min faster for a dough containing the combination as defined in any one of claims 1-2 than that obtained for dough with 6% or 14% sugar without the combination as defined in any one of claims 1-3.

12. A process according to any one of claims 3-11, wherein the mixing step and the proofing step involve the action of the thermo-labile alpha-amylase as defined in claim 1 on starch polysaccharides in the flour to produce fermentable sugar.

13. A process according to any one of claims 3-12, wherein proofing step involves the action of the thermo-stable amyloglucosidase as defined in claim 1 and the maltogenic amylase as defined in claim 1 on poly-, oligo- and/or di-saccharides in the dough to increase the content of glucose and maltose in the baked product.

14. A process according to any one of claims 4, 6, 9, wherein the dough is baked at a temperature in a range of from 180 to 250 °C, and a baked product is obtained having a fructose content of at the most 1% by weight.

15. A process according to any one of claims 4, 6, 9, wherein the dough is baked and a baked product is obtained comprising
i) fructose in a concentration of at the most 2.6% by weight such as at the most 2.0% by weight or about 1% by weight,
ii) glucose in a concentration range from about 1.5 to about 4.5% by weight,
iii) lactose in a concentration of at the most 0.5% by weight,
iv) maltose in a concentration range of from 2.5 to 5.5% by weight,
v) saccharose in a concentration of at the most 0.5% by weight,
wherein the concentration is based on the total weight of the baked product.

16. A combination according to claim 2, wherein the bakery product is a baked product that has a fructose content of 1% w/w or less.

## Patentansprüche

1. Kombination, bestehend aus einer Alpha-Amylase (EC 3.2.1.1), die thermolabil ist und Aktivität in einem Bereich von 30 bis 65 °C aufweist, einer Amyloglucosidase (EC 3.2.1.3), die thermostabil ist und optimale Aktivität bei etwa 60-65 °C aufweist, und einer maltogenen Amylase (EC 3.2.1.133), die optimale Aktivität in einem Bereich von 50 bis 75 °C aufweist, und wobei die thermolabile Alpha-Amylase, die thermostabile Amyloglucosidase und die maltogene Amylase in Verhältnissen vorliegen, die Folgendem entsprechen
von 20 bis 48 Fau oder von 28 bis 40 Fau der thermolabilen Alpha-Amylase,
von 578 bis 1650 Agu oder von 660 bis 1650 Agu der thermostabilen Amyloglucosidase, und
von 500 bis 2500 Manu oder von 1000 bis 2500 Manu der maltogenen Amylase.

2. Kombination nach Anspruch 1 zur Verwendung bei der Zubereitung eines Backprodukts ohne Zuckerzusatz.

3. Prozess zur Herstellung eines Backprodukts ohne Zuckerzusatz, wobei der Prozess umfasst
i) Mischen von Mehl mit einem Gehalt an geschädigter Stärke von mindestens 5 Gew.-% mit einer Kombination wie in einem der Ansprüche 1-2 definiert; einer Hefe; Wasser und gegebenenfalls anderen für die Zubereitung eines Teigs üblichen Zutaten, um einen Teig zu erhalten,
ii) Gehenlassen des Teigs,
iii) gegebenenfalls Formen des Teigs in eine gewünschte Form,
iv) gegebenenfalls Einfrieren des ungebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C, und
v) gegebenenfalls Vorbacken oder Backen des gegebenenfalls geformten Teigs bei einer Temperatur in einem Bereich von 180 bis 250 °C.

4. Prozess nach Anspruch 3, umfassend
i) Mischen von Mehl mit einem Gehalt an geschädigter Stärke von mindestens 5 Gew.-% mit einer Kombination wie in einem der Ansprüche 1-3 definiert; einer Hefe; Wasser und gegebenenfalls anderen für die Zubereitung eines Teigs üblichen Zutaten, um einen Teig zu erhalten,
ii) Gehenlassen des Teigs,
iii) gegebenenfalls Formen des Teigs in eine gewünschte Form,
iv) gegebenenfalls Einfrieren des ungebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C, und
v) Vorbacken oder Backen des gegebenenfalls geformten Teigs bei einer Temperatur in einem Bereich von 180 bis 250 °C.

5. Prozess nach Anspruch 3, umfassend
i) Mischen von Mehl mit einem Gehalt an geschädigter Stärke von mindestens 5 Gew.-% mit einer Kombination wie in einem der Ansprüche 1-2 definiert; einer Hefe; Wasser und gegebenenfalls anderen für die Zubereitung eines Teigs üblichen Zutaten, um einen Teig zu erhalten,
ii) Gehenlassen des Teigs,
iii) gegebenenfalls Formen des Teigs in eine gewünschte Form,
iv) gegebenenfalls Einfrieren des ungebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C, und
v) Vorbacken des gegebenenfalls geformten Teigs bei einer Temperatur in einem Bereich von 180 bis 250 °C, gefolgt von
Einfrieren des vorgebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C.

6. Prozess nach Anspruch 3, umfassend
i) Mischen von Mehl mit einem Gehalt an geschädigter Stärke von mindestens 5 Gew.-% mit einer Kombination wie in einem der Ansprüche 1-2 definiert; einer Hefe; Wasser und gegebenenfalls anderen für die Zubereitung eines Teigs üblichen Zutaten, um einen Teig zu erhalten,
ii) Gehenlassen des Teigs,
iii) Formen des Teigs in eine gewünschte Form,
iv) gegebenenfalls Einfrieren des ungebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C, und
v) Backen des gegebenenfalls geformten Teigs bei einer Temperatur in einem Bereich von 180 bis 250 °C.

7. Prozess nach Anspruch 3, umfassend
i) Mischen von Mehl mit einem Gehalt an geschädigter Stärke von mindestens 5 Gew.-% mit einer Kombination wie in einem der Ansprüche 1-2 definiert; einer Hefe; Wasser und gegebenenfalls anderen für die Zubereitung eines Teigs üblichen Zutaten, um einen Teig zu erhalten,
ii) Gehenlassen des Teigs,
iii) gegebenenfalls Formen des Teigs in eine gewünschte Form,
iv) Einfrieren des ungebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C, und
v) gegebenenfalls Vorbacken oder Backen des gegebenenfalls geformten Teigs bei einer Temperatur in einem Bereich von 180 bis 250 °C.

8. Prozess nach Anspruch 3, umfassend
i) Mischen von Mehl mit einem Gehalt an geschädigter Stärke von mindestens 5 Gew.-% mit einer Kombination wie in einem der Ansprüche 1-2 definiert; einer Hefe; Wasser und gegebenenfalls anderen für die Zubereitung eines Teigs üblichen Zutaten, um einen Teig zu erhalten,
ii) Gehenlassen des Teigs,
iii) Formen des Teigs in eine gewünschte Form,
iv) Einfrieren des ungebackenen Produkts bei einer Temperatur in einem Bereich von -35 °C - -45 °C für einen Zeitraum von etwa 15 Minuten bis etwa 30 Minuten, gefolgt von Gefrieren bei etwa -18 °C, und
v) gegebenenfalls Vorbacken oder Backen des gegebenenfalls geformten Teigs bei einer Temperatur in einem Bereich von 180 bis 250 °C.

9. Prozess nach Anspruch 3, umfassend
a) Mischen von Mehl mit einem Gehalt an geschädigter Stärke von mindestens 5 Gew.-% mit einer Kombination wie in einem der Ansprüche 1-2 definiert; einer Hefe; Wasser und gegebenenfalls anderen für die Zubereitung eines Teigs üblichen Zutaten, um einen Teig zu erhalten,
b) Gehenlassen des Teigs,
c) Backen des Teigs bei einer Temperatur in einem Bereich von 180 bis 250 °C.

10. Prozess nach einem der Ansprüche 3-9, wobei die Gehzeit, um eine bestimmte Höhe des Teigs zu erhalten, im Vergleich zur Gehzeit eines Teigs auf die gleiche bestimmte Höhe um 30 % bis 50 % reduziert wird, wobei der Vergleichsteig abgesehen von Weglassung der Kombination wie in einem der Ansprüche 1-3 definiert und abgesehen von Einbeziehung von 6-7 % Zucker die gleichen Zutaten aufweist.

11. Prozess nach einem der Ansprüche 3-10, wobei die Gehzeit, um im Vergleich zum Startwert die doppelte Höhe zu erhalten, bei einem Teig, der die Kombination wie in einem der Ansprüche 1-2 definiert enthält, mindestens 15 Minuten schneller, wie etwa 15 bis 30 Minuten schneller ist als die, die bei Teig mit 6 % oder 14 % Zucker ohne die Kombination wie in einem der Ansprüche 1-3 definiert erhalten wird.

12. Prozess nach einem der Ansprüche 3-11, wobei der Schritt des Mischens und der Schritt des Gehenlassens das Wirken der thermolabilen Alpha-Amylase wie in Anspruch 1 definiert auf Stärkepolysaccharide im Mehl beinhaltet, um fermentierbaren Zucker zu erzeugen.

13. Prozess nach einem der Ansprüche 3-12, wobei der Schritt des Gehenlassens das Wirken der thermostabilen Amyloglucosidase wie in Anspruch 1 definiert und der maltogenen Amylase wie in Anspruch 1 definiert auf Poly-, Oligo- und/oder Disaccharide im Teig beinhaltet, um den Gehalt an Glucose und Maltose im gebackenen Produkt zu erhöhen.

14. Prozess nach einem der Ansprüche 4, 6, 9, wobei der Teig bei einer Temperatur in einem Bereich von 180 bis 250 °C gebacken wird und ein gebackenes Produkt erhalten wird, das einen Fructosegehalt von höchstens 1 Gew.-% aufweist.

15. Prozess nach einem der Ansprüche 4, 6, 9, wobei der Teig gebacken wird und ein gebackenes Produkt erhalten wird, das Folgendes umfasst
i) Fructose in einer Konzentration von höchstens 2,6 Gew.-%, wie etwa höchstens 2,0 Gew.-% oder etwa 1 Gew.-%,
ii) Glucose in einem Konzentrationsbereich von etwa 1,5 bis etwa 4,5 Gew.-%,
iii) Lactose in einer Konzentration von höchstens 0,5 Gew.-%,
iv) Maltose in einem Konzentrationsbereich von 2,5 bis 5,5 Gew.-%,
v) Saccharose in einer Konzentration von höchstens 0,5 Gew.-%,
wobei die Konzentration auf das Gesamtgewicht des gebackenen Produkts bezogen ist.

16. Kombination nach Anspruch 2, wobei das Backprodukt ein gebackenes Produkt ist, das einen Fructosegehalt von 1 % w/w oder weniger aufweist.

## Revendications

1. Combinaison composée d'une alpha-amylase (EC 3.2.1.1), qui est thermolabile et présente une activité dans une plage de 30 à 65 °C, d'une amyloglucosidase (EC 3.2.1.3), qui est thermostable et présente une activité optimale à une température d'environ 60-65 °C, et une amylase maltogène (EC 3.2.1.133), qui présente une activité optimale dans une plage de 50 à 75 °C, et dans laquelle l'alpha-amylase thermolabile, l'amyloglucosidase thermostable et l'amylase maltogène sont présents dans des ratios correspondant à
de 20 à 48 Fau ou de 28 à 40 Fau de l'alpha-amylase thermolabile,
de 578 à 1650 Agu ou de 660 à 1650 Agu de l'amyloglucosidase thermostable, et
de 500 à 2500 Manu ou de 1000 à 2500 Manu de l'amylase maltogène.

2. Combinaison selon la revendication 1 destinée à être utilisée dans la préparation d'un produit de boulangerie sans sucre ajouté.

3. Processus de production d'un produit de boulangerie sans sucre ajouté, le processus comprenant les étapes consistant à
i) mélanger de la farine avec une teneur en amidon endommagé d'au moins 5 % en poids avec une combinaison telle que définie dans l'une quelconque des revendications 1-2 ; une levure ; de l'eau, et facultativement d'autres ingrédients courants pour préparer une pâte, afin d'obtenir une pâte,
ii) faire lever la pâte,
iii) facultativement mettre la pâte en forme selon une forme souhaitée,
iv) facultativement, geler le produit non cuit à une température dans une plage allant de -35 °C à -45 °C pendant une période de temps d'environ 15 min à environ 30 min, puis congeler à environ -18 °C, et
v) facultativement précuire ou cuire la pâte facultativement mise en forme à une température comprise dans une plage allant de 180 à 250 °C.

4. Processus selon la revendication 3 comprenant les étapes consistant à
i) mélanger de la farine avec une teneur en amidon endommagé d'au moins 5 % en poids avec une combinaison telle que définie dans l'une quelconque des revendications 1-3 ; une levure ; de l'eau, et facultativement d'autres ingrédients courants pour préparer une pâte, afin d'obtenir une pâte,
ii) faire lever la pâte,
iii) facultativement mettre la pâte en forme selon une forme souhaitée,
iv) facultativement, geler le produit non cuit à une température dans une plage allant de -35 °C à -45 °C pendant une période de temps d'environ 15 min à environ 30 min, puis congeler à environ -18 °C, et
v) précuire ou cuire la pâte facultativement mise en forme à une température comprise dans une plage allant de 180 à 250 °C.

5. Processus selon la revendication 3, comprenant les étapes consistant à
i) mélanger de la farine avec une teneur en amidon endommagé d'au moins 5 % en poids avec une combinaison telle que définie dans l'une quelconque des revendications 1-2 ; une levure ; de l'eau, et facultativement d'autres ingrédients courants pour préparer une pâte, afin d'obtenir une pâte,
ii) faire lever la pâte,
iii) facultativement mettre la pâte en forme selon une forme souhaitée,
iv) facultativement, geler le produit non cuit à une température dans une plage allant de -35 °C à -45 °C pendant une période de temps d'environ 15 min à environ 30 min, puis congeler à environ -18 °C, et
v) précuire la pâte facultativement mise en forme à une température comprise entre 180 et 250 °C, puis
geler le produit non cuit à une température dans une plage allant de -35 °C à -45 °C pendant une période de temps d'environ 15 min à environ 30 min, puis congeler à environ -18 °C.

6. Processus selon la revendication 3 comprenant les étapes consistant à
i) mélanger de la farine avec une teneur en amidon endommagé d'au moins 5 % en poids avec une combinaison telle que définie dans l'une quelconque des revendications 1-2 ; une levure ; de l'eau, et facultativement d'autres ingrédients courants pour préparer une pâte, afin d'obtenir une pâte,
ii) faire lever la pâte,
iii) mettre la pâte en forme selon une forme souhaitée,
iv) facultativement, geler le produit non cuit à une température dans une plage allant de -35 °C à -45 °C pendant une période de temps d'environ 15 min à environ 30 min, puis congeler à environ -18 °C, et
v) cuire la pâte facultativement mise en forme à une température comprise dans une plage allant de 180 à 250 °C.

7. Processus selon la revendication 3 comprenant les étapes consistant à
i) mélanger de la farine avec une teneur en amidon endommagé d'au moins 5 % en poids avec une combinaison telle que définie dans l'une quelconque des revendications 1-2 ; une levure ; de l'eau, et facultativement d'autres ingrédients courants pour préparer une pâte, afin d'obtenir une pâte,
ii) faire lever la pâte,
iii) facultativement mettre la pâte en forme selon une forme souhaitée,
iv) geler le produit non cuit à une température dans une plage allant de -35 °C à -45 °C pendant une période de temps d'environ 15 min à environ 30 min, puis congeler à environ -18 °C, et
v) facultativement précuire ou cuire la pâte facultativement mise en forme à une température comprise dans une plage allant de 180 à 250 °C.

8. Processus selon la revendication 3 comprenant les étapes consistant à
i) mélanger de la farine avec une teneur en amidon endommagé d'au moins 5 % en poids avec une combinaison telle que définie dans l'une quelconque des revendications 1-2 ; une levure ; de l'eau, et facultativement d'autres ingrédients courants pour préparer une pâte, afin d'obtenir une pâte,
ii) faire lever la pâte,
iii) mettre la pâte en forme selon une forme souhaitée,
iv) geler le produit non cuit à une température dans une plage allant de -35 °C à -45 °C pendant une période de temps d'environ 15 min à environ 30 min, puis congeler à environ -18 °C, et
v) facultativement précuire ou cuire la pâte facultativement mise en forme à une température comprise dans une plage allant de 180 à 250 °C.

9. Processus selon la revendication 3 comprenant les étapes consistant à
a) mélanger de la farine avec une teneur en amidon endommagé d'au moins 5 % en poids avec une combinaison telle que définie dans l'une quelconque des revendications 1-2 ; une levure ; de l'eau, et facultativement d'autres ingrédients courants pour préparer une pâte, afin d'obtenir une pâte,
b) faire lever la pâte,
c) cuire la pâte à une température comprise dans une plage allant de 180 à 250 °C.

10. Processus selon l'une quelconque des revendications 3-9, dans lequel le temps de levée pour obtenir une hauteur spécifiée de la pâte est réduit de 30 % à 50 % par rapport au temps de levée d'une pâte jusqu'à la même hauteur spécifiée, dans lequel la pâte de comparaison contient les mêmes ingrédients, à l'exception de l'omission de la combinaison telle que définie dans l'une quelconque des revendications 1-3 et à l'exception de l'inclusion de 6-7 % de sucre.

11. Processus selon l'une quelconque des revendications 3-10, dans lequel le temps de levée pour obtenir deux fois la hauteur par comparaison à la valeur de départ est plus rapide d'au moins 15 minutes, par exemple plus rapide d'environ 15 à 30 minutes pour une pâte contenant la combinaison telle que définie dans l'une quelconque des revendications 1-2 que celui obtenu pour une pâte avec 6 % ou 14 % de sucre sans la combinaison telle que définie dans l'une quelconque des revendications 1-3.

12. Processus selon l'une quelconque des revendications 3-11, dans lequel l'étape de mélange et l'étape de levée impliquent l'action de l'alpha-amylase thermolabile telle que définie dans la revendication 1 sur des polysaccharides d'amidon présents dans la farine pour produire du sucre fermentescible.

13. Processus selon l'une quelconque des revendications 3-12, dans lequel l'étape de levée implique l'action de l'amyloglucosidase thermostable telle que définie dans la revendication 1 et de l'amylase maltogène telle que définie dans la revendication 1 sur des poly-, oligo- et/ou disaccharides dans la pâte pour augmenter la teneur en glucose et en maltose dans le produit cuit.

14. Processus selon l'une quelconque des revendications 4, 6, 9, dans lequel la pâte est cuite à une température comprise dans une plage allant de 180 à 250 °C, et un produit cuit est obtenu, présentant une teneur en fructose d'au plus 1 % en poids.

15. Processus selon l'une quelconque des revendications 4, 6, 9, dans lequel la pâte est cuite et un produit cuit est obtenu, comprenant
i) du fructose à une concentration d'au plus 2,6 % en poids, telle qu'au plus 2,0 % en poids ou environ 1 % en poids,
ii) du glucose dans une plage de concentration allant d'environ 1,5 à environ 4,5 % en poids,
iii) du lactose à une concentration d'au plus 0,5 % en poids,
iv) du maltose dans une plage de concentration allant de 2,5 à 5,5 % en poids,
v) du saccharose à une concentration d'au plus 0,5 % en poids,
dans lequel la concentration est basée sur le poids total du produit cuit.

16. Combinaison selon la revendication 2, dans laquelle le produit de boulangerie est un produit cuit qui présente une teneur en fructose de 1 % p/p ou moins.
